# EUROPEAN PATENT SPECIFICATION

(11) **EP 2 462 451 B1**
(45) Date of publication and mention of the grant of the patent: **17.02.2016**
(21) Application number: 10745535.4
(22) Date of filing: 04.08.2010
(51) Int. Cl.: G01N 33/569

(54) **HUMAN HCV-INTERACTING PROTEINS AND METHODS OF USE**
MIT HCV INTERAGIERENDE HUMANE PROTEINE UND METHODEN FÜR DEREN VERWENDUNG
PROTÉINES HUMAINES INTERAGISSANT AVEC VHC ET PROCÉDÉS D'UTILISATION

(30) Priority: 05.08.2009 EP 09167303
(43) Date of publication of application: 13.06.2012
(73) Proprietor: Nexigen GmbH, 50829 Köln (DE)
(72) Inventor: CARL, Claudia, 53175 Bonn (DE); HENNEMANN, Hanjo, 41470 Neuss (DE)
(74) Representative: Vossius & Partner Patentanwälte Rechtsanwälte mbB
(86) International application number: PCT/EP2010/004932
(87) International publication number: WO 2011/015379

(56) References cited:
- WO-A1-2009/127693
- WO-A2-2008/025564
- US-A1- 2003 215 803
- US-A1- 2007 105 114
- US-A1- 2007 105 133
- DE CHASSEY B ET AL: "Hepatitis C virus infection protein network", MOLECULAR SYSTEMS BIOLOGY, vol. 4, November 2008 (2008-11), XP002605972,
- BOROWSKI PETER ET AL: "Protein kinase C recognizes the protein kinase A-binding motif of nonstructural protein 3 of hepatitis C virus", JOURNAL OF BIOLOGICAL CHEMISTRY, vol. 274, no. 43, 22 October 1999 (1999-10-22), pages 30722-30728, XP002605973, ISSN: 0021-9258 cited in the application
- GUIMARAES DENISE P ET AL: "Interferon-inducible guanylate binding protein (GBP)-2: A novel p53-regulated tumor marker in esophageal squamous cell carcinomas", INTERNATIONAL JOURNAL OF CANCER, vol. 124, no. 2, January 2009 (2009-01), pages 272-279, XP002605974, ISSN: 0020-7136

## Description

### FIELD OF THE INVENTION

The present invention generally relates to the technical field of molecular biology and virology, respectively, and the development of antiviral drugs in particular. In one aspect, the present invention relates to a method for identifying and cloning nucleic acid molecules encoding proteins or fragments thereof, capable of interacting with proteins associated with the Human Hepatitis C virus (HCV) and thus being suitable either alone or in complex with the HCV protein for serving as a target for the development of antiviral drugs. The invention is hence also in the field of drug development, therapeutics but also diagnostics.

### BACKGROUND OF THE INVENTION

An estimated 170 million people are infected with the Hepatitis C virus (HCV) worldwide and frequently these infections result in cirrhosis and hepatocellular carcinoma in long-term chronically infected cells.

HCV is an enveloped, positive stranded RNA virus and a member of the Flaviviridae family, which includes three genera: Flavivirus, Pestivirus and Hepacivirus. Exposure to HCV results in an overt acute disease in a small percentage of cases, while in most instances the virus establishes a chronic infection causing liver inflammation and slowly progresses into liver failure and cirrhosis. (Warson, FEMS Microbiol. Rev., 74:201-204, 1994.). Epidemiological surveys indicate HCV plays an important role in hepatocellular carcinoma pathogenesis (Kew, FEMS Microbiol. Rev., 14:211-220, 1994, Alter, Blood, §5:1681-1695, 1995.).

The HCV genome consists of a single strand RNA about 9.5 kb in length, encoding a precursor polyprotein about 3000 amino acids, which is subsequently processed by cellular and viral proteases into the structural and non-structural proteins (Glenn et al., Molecular virology of the hepatitis C virus: implication for the novel therapies. Clin Liver Dis. 9, 353-369 2005, Choo et al, Science, 244:359-362, 1989, Takamizawa et al. J. Virol, 65:1105-1113, 1991.).

The current treatment of HCV infection involves 6-12 months of combination therapy with pegylated interferon with an extended half-life and ribavirin. Ribavarin is known to inhibit inosine-5'-monophosphate dehydrogenase (IMPDH), a cellular enzyme that catalyzes a crucial step in the biosynthesis of guanine nucleotides and presumably reduces the cellular pool preferentially required for viral replication (Yupeng et al., Drug Discovery Today,12, 209-217, 2007). However, most individuals infected with genotype 1, which is one of the most relevant genotypes, will not experience long term viral clearance with combination therapy. This draws attention to the urgent need for the development of novel drugs. Viral proteins as well as host factors can be used as targets for the development of drugs. Many of the current antiviral drugs are designed to function as specific viral inhibitors, by inhibiting virus encoded enzymes essential for viral replication. However, those drugs suffer from many drawbacks. First, they can only be used to treat specific viral species, or even only certain subtypes of a viral species. This narrow spectrum of action, although reducing the potential for toxicity associated with targeting nonspecific enzymes, greatly limits the usage and market potential of each of these drugs. In addition, many viruses are able to mutate, evolve and at high rates generate drug resistant, mutant viral species, further limiting the usefulness of viral inhibitors. One group of known anti-HCV targets for drug development are the viral enzymes NS3-4A serine protease and NS5B RNA dependent RNA polymerase, which are essential for viral replication. The protein NS3 is a multifunctional protein with several associated enzymatic activities. The c-terminal 450 amino acids constitute a polynucleotides-stimulated NTPase activity (Jin, L., and D. L. Peterson. 1995), 3'-5' unwinding activity (Gwack et al., Biochem Biophys. Res. Commun. Aug 14;225(2): 654-9 1996), and a single stranded RNA binding activity (Tai, C. L., W. K. Chi, D. S. Chen, and L. H. Hwang. J Virol. 70(12):8477-84, 1996).

The N-terminal third of NS3 is a chymotrypsine-like serine protease. The serine protease activity is dependent upon an interaction with NS4A cofactor and is responsible for the processing of the nonstructural proteins at the NS3-NS4A, NS4A-NS4B, NS4B-NS5A and NS5A-NS5B junction. Viral proteins and genomes specifically interact with and recruit certain molecules or pathways to facilitate viral genome replication and protein translation as well as the activation of the host immune response. The NS3/4A protease has been shown to modulate innate cellular antiviral responses by cleaving two cellular adaptor proteins, LMP1 and MECL-1, which have been shown to modulate innate cellular antiviral response through the generation of MHC class I ligands (Ling et al., Biochem. J.,384, 401-409 2004). Another role of NS3 in intracellular transport and secretion through its binding to ELKS, a protein family which is involved in intracellular transport and secretory pathways has been reported (Hidajat et al., Journal of General Virology, 86, 2197-2208, 2005). The helicase domain of NS3 was shown to interact with protein kinase A (PKA; Aoubala et al., Journal Gene Virology 82, 1637-46, 2001; Borowski et al., J Biol Chem. 274:30722-8 1999) and can act as an inhibitor of PKA. However few inhibitors of the HCV helicase have been explored as antivirals because less is known about the mechanism of action of the HCV helicase.

It is an object of the present invention to provide for human proteins capable of interacting with the HCV NS3 protein or portions thereof.

Assays for drug development are a further object of the present invention as well as diagnostic methods for HCV detection and load determination.

### SUMMARY OF THE INVENTION

The present invention relates to novel drug targets useful in the treatment of viral diseases. More particularly, the present invention is directed to human MARCH7 interacting with the HCV NS3 protein and drugs interfering with said interactions. Experiments performed in accordance with the present invention surprisingly revealed MARCH7 is interacting with a viral NS3 protein. Accordingly, the so identified proteins as well as protein-protein interaction and complex formation between HCV NS3 and the human MARCH7, respectively, provide suitable targets for therapeutic intervention and design of agents, capable of modulating the same. In this context, the present invention provides both, nucleic acid molecules encoding MARCH7 with the viral disease-associated protein and the proteins encoded by said nucleic acid molecules or obtainable according to the methods of the present invention as well as complexes comprising the viral disease-associated protein and the host (human) protein identified by the method of the present invention.

Furthermore, the present application discloses an antibody that specifically binds either to the complex of the HCV protein and the human protein or to the binding domain of the HCV protein and the human protein, respectively, or it binds to a protein which by way of amino acid substitution, deletion and/or addition is no longer capable of forming a respective complex with NS3.

The present invention also concerns a method for screening compounds, capable of modulating a particular protein-protein interaction and complex formation and/or stability, respectively. Also disclosed are compounds obtainable according to said method and the use of said compounds for the preparation of a medicament for the treatment of HCV induced diseases. For example, appropriate antiviral drugs maybe derived from the human protein identified as protein binding partner of the HCV accessory protein by way of identifying the binding domain and designing corresponding peptides or mimetics thereof, capable of interfering with the interaction of the native protein with the HCV NS3 protein.

### BRIEF DESCRIPTION OF THE DRAWINGS

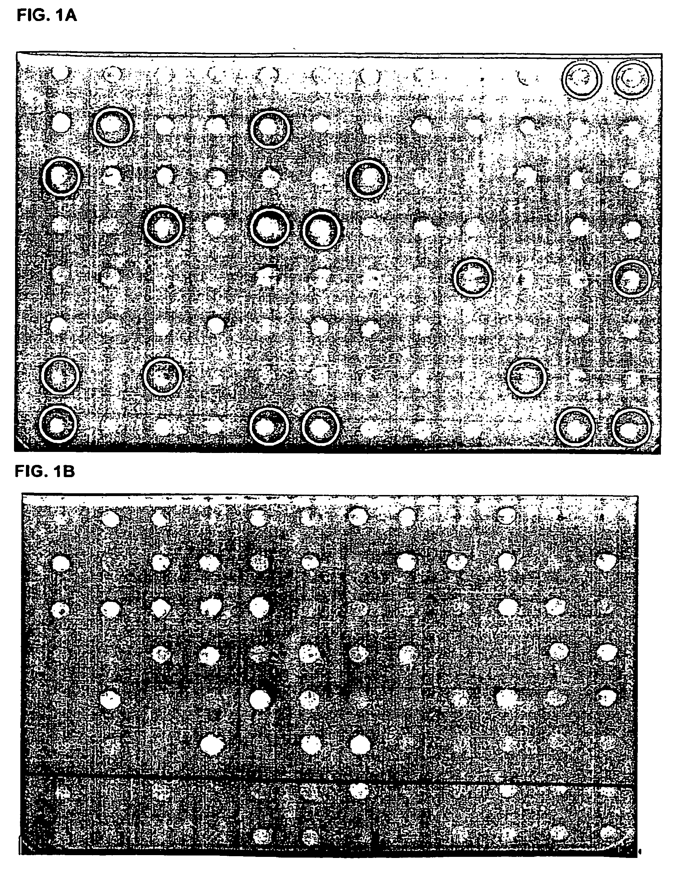

- FIG. 1:: Figure 1 illustrates the identification of NS3 FL or NS3 HCF protein complexes, which complement growth defects of a temperature sensitive Ras mutant at the restrictive temperature of 37°C. A first specificity test shows the dependency of cell growth on library protein expression and eliminates false positive clones during the screening procedure. Interacting library proteins, located at the membrane recruit NS3-Sos fusion proteins and thereby activate the Ras signaling pathway.
In particular Figure 1A represents growth of clones expressing NS3 FL or NS3 HCF and a protein from a hepatocyte library. Library proteins are under the control of an inducible promoter. A selection of 95 yeast clones and one control clone are shown under inducible conditions. Figure 1B represents the same clones; however the expression of the library protein is shut off by the addition of glucose (Glucose 37°C, Figure 1B). Clones were scored positive only when growth signal is dependent on the expression of library protein (clones were marked by circles, FIG.1A).
Positive control is indicated by a circle; see bottom right of Figure 1A.

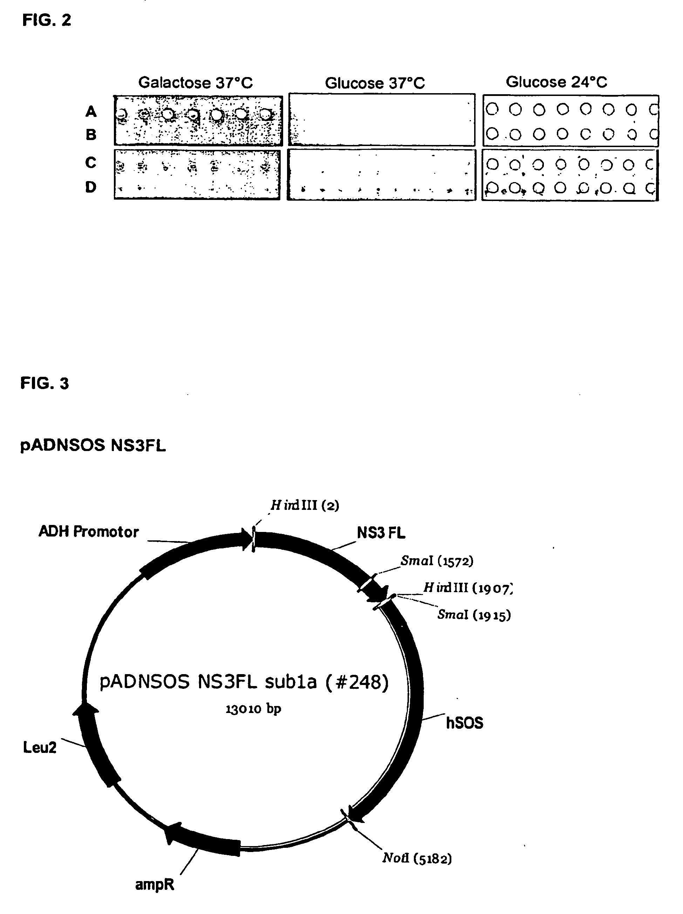

- FIG. 2:: Figure 2 shows the microbial growth response resulting from complexes of NS3FL or NS3 HCF protein and different host factors identified according to the method of the present invention. Binding specificity of the host proteins to NS3 HCF or NS3 FL is also demonstrated. Growth of yeast cells at the restrictive temperature of 37°C is dependent on the expression of host factors as well as of the interacting target protein NS3 HCF, respectively NS3FL, since only upon the interaction of the host proteins with NS3 the effector responsible for cell growth is recruited to the cell membrane. Vif was used as a negative control target protein; see table below. Row A: GBP2 + SOS-NS3FL; row B: GBP2 + Sos-Vif; row C: MARCH7 + SOS-NS3 HCF; row D: MARCH7 + SOS-Vif.
No binding is to be expected to Vif, therefore no cell growth is detected. Fig.2 shows the host proteins identified according to the method of the present invention and sequenced after the respective and specific interaction with NS3 was demonstrated. Under screening conditions (column galactose 37°C) the expression of the host factors is induced and growth is dependent on their interaction with the NS3 fusion protein. As can be seen, host factors mediate growth only in combination with the NS3 fusion protein, but not when combined with the heterologous Vif fusion protein. The second column (glucose 37°C) indicates host factor dependent growth, since repression of the host factor expression (glucose represses GAL1 promotor driven expression cassette) abolished the growth response in all cases. The right column of Fig. 2 represents cell growth controls at the non-restrictive temperature (24°C).
- FIG. 3:: Figure 3 schematically represents vector pADNSos-NS3FL, used for the expression of the first fusion protein for use in the method of the present invention. The sites of ADH promoter, NS3 and SOS are indicated.

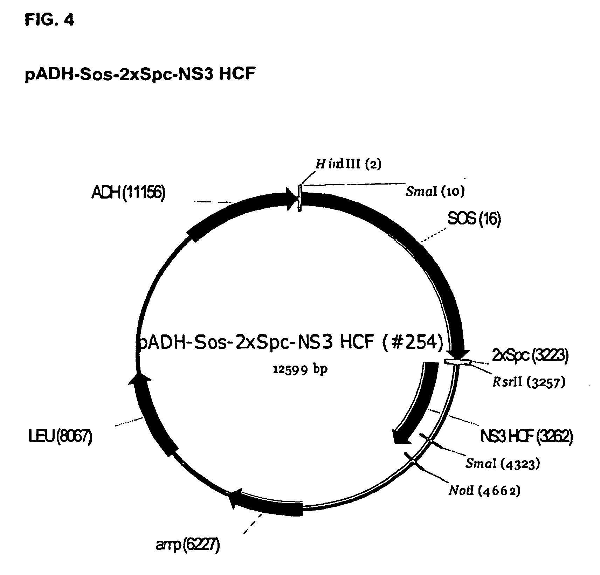

- FIG. 4:: Figure 4 schematically represents vector pADH Sos-2xSpc-NS3 HCF, used for the expression of the first fusion protein in the method of the present invention. The sites of ADH promoter, NS3 HCF and Sos are indicated.

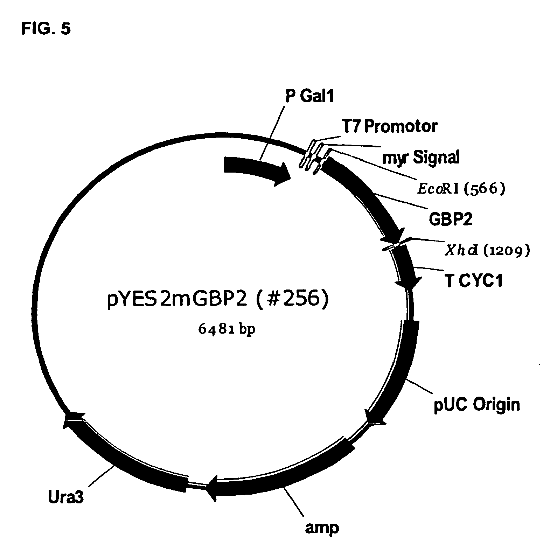

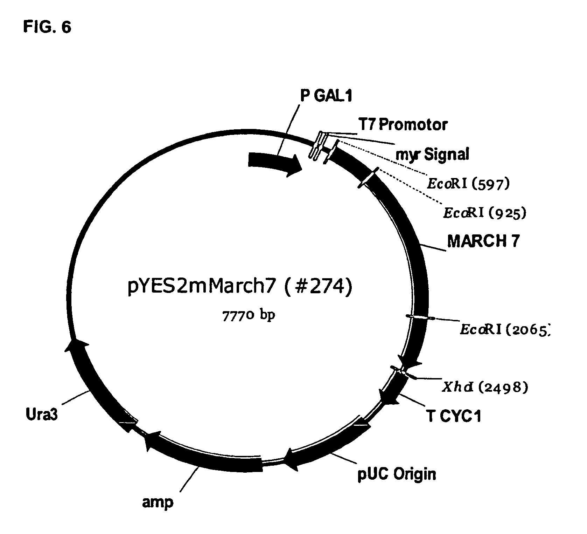

- FIG. 5+6:: Figure 5 and 6 schematically represent pmyr vectors used for the expression of the second fusion protein for use in the method of the present invention. The sites of the (inducible) GAL1 promoter, the myristoylation signal and the second protein (from hepatocyte library) are indicated.

### Definitions

"cDNA", as the term is used herein, generally describes complementary DNA, *i.e.* a piece of DNA lacking internal, non-coding segments (introns) and transcriptional regulatory sequences. A cDNA may further contain un-translated regions (UTRs) that are responsible for translational control in the corresponding RNA molecule. cDNA can be produced using various methods, such as synthesis in the laboratory by reverse transcription from messenger RNA extracted from cells.

"Infection", as the term is used herein, generally relates to the entry, replication, insertion, lysis or other event or process involved with the pathogenesis of a virus into a host cell. Thus, decreasing infection includes decreasing entry, replication, insertion, lysis, or other pathogenesis of a virus into a cell or subject, or combinations thereof. Infection includes the introduction of an infectious agent, such as a non-recombinant virus, recombinant virus, plasmid, or other agent capable of infecting a host, such as the cell of a subject.

"Mimetic", as the term is used herein, generally refers to a molecule that mimics the activity of an agent. The term "mimetics" when used in the context of peptides, refers to molecular structures, which serve as substitutes for the peptides of the present invention in the interaction with HCV NS3. Peptide mimetics, as used herein, include synthetic structures which may or may not contain amino acids and/or peptide bonds, but retain the structural and functional features of a peptide ligand. The term "peptide mimetics" also includes peptides and oligopeptides, which are peptides or oligomers of N-substituted amino acids. Further included as peptide mimetics are peptide libraries, which are collections of peptides designed to be of a given amino acid length and representing all conceivable sequences of amino acids corresponding thereto.

"Operably linked", as the term is used herein, generally describes a first nucleic acid sequence as operably linked with a second nucleic acid sequence when the first nucleic acid sequence is placed in a functional relationship with the second one. For instance, a promoter is operably linked to a coding sequence if the promoter affects the transcription or expression of said coding sequence. Generally, operably linked DNA sequences are contiguous and, where necessary, to join two proteins coding regions, in the same reading frame.

"Pharmaceutical agent or drug", as the terms are used herein, generally relate to a chemical compound or composition capable of inducing a desired therapeutic or prophylactic effect when administered to a subject, alone or in combination with one or more therapeutic agent(s) or pharmaceutically acceptable carriers.

"Preventing" a disease, as the term is used herein, generally refers to the inhibition of the full development of a disease, for example the prevention of development of a viral infection.

"Treatment", as the term is used herein, generally describes a therapeutic intervention that ameliorates a sign or symptom of a disease or pathological condition related to, for example, a HCV infection, such as inhibiting or decreasing HCV infection.

"First fusion protein", as the term is used herein, generally relates to a protein comprising an effector and a "target" protein such as an HCV protein. A target protein generally is a known protein that is being examined as to whether it can be involved in a protein-protein interaction. Preferably, the first fusion protein of the present invention comprises a peptide linker molecule, arranged between the effector and the target protein.

"Second fusion protein", as the term is used herein, generally relates to a protein comprising a cell compartment localizing domain and a second protein such as a human protein, which can bind the target protein or is suspected of being able to bind to the target protein.

"Effector", as the term is used herein, generally describes a peptide or polypeptide that can be expressed as a fusion protein and, when so expressed, can activate a reporter molecule, provided the effector protein is translocated to the cell compartment containing the reporter molecule. However, also an active fragment of an effector such as a guanine exchange factor (GEF) can be used to practice the invention, provided the active fragment comprises a sufficient portion of the effector so as to confer the effector function. Such active fragments of an effector are considered to be within the meaning of the term "effector" as used herein.

"Candidate protein", as the term is used herein, generally relates to the protein which is suspected for binding to the HCV target protein. The term "candidate protein" may be used interchangeably with the terms "host protein" and "human protein". According to the present invention, the term "candidate protein" does not only include full-length protein, but also comprises parts of the protein for example oligopeptides and peptides, respectively.

As used herein, a composition refers to any mixture. It can be a solution, a suspension, liquid, powder, a paste, aqueous, non-aqueous or any combination thereof.

The term "derivative" as applied to a protein or a peptide chain herein means a compound wherein one or more of the amino, hydroxyl, or carboxyl groups in a side chain of the peptide, or the terminal amino or carboxyl groups, are modified to a derivative functional group. An amino group may be derivatized as an amide (such as an alkylcarboxamide, acetamide), a carbamate (such as an alkyl carbamate, *e.g.* methyl carbamate or t-butylcarbamate), or an urea. A hydroxyl group may be derivatized as an ester (such as an alkanoate, *e.g.* acetate, propionate, or all arenecarboxylate, *e.g.* benzoate), a carbamate (such as an alkyl carbamate, *e.g.* methyl carbamate), a carbonate (such as an alkyl carbonate, *e.g.* ethyl carbonate. A carboxyl group may be derivatized as an ester (such as an alkyl ester, *e.g.* ethyl ester) or an amide (*e.g.* primary carboxamide, an N-alkyl secondary carboxamide, or an N,N-dialkylcarboxamide). The person skilled in the art will appreciate that derivatives of the peptide will be expected to result in retention of the properties of the parent peptide, either because the incorporation of the derivative group does not change the properties of the peptide, or the derivatizing group is removed *in vivo* (*e.g.* via metabolism). Preferred embodiments of the invention are those wherein three or fewer of the amino, carboxyl, and hydroxyl groups, and preferably two or fewer, or one or none, are modified to a derivative functional group. The term "derivative" also includes salts, includes salts of derivatives. Derivatives may include terminal derivatives.

The term "terminal derivative" used in reference to a peptide means a peptide where the C-terminal carboxylate group, or the N-terminal amino group, or both is modified to a derivative functional group. The C-terminal carboxyl group may be derivatized as an ester (such as an alkyl ester, *e.g.* ethyl ester) or an amide (*e.g.* primary carboxamide, an N-alkyl secondary carboxamide, or an N,N-dialkylcarboxamide). The N-terminal amino group may be derivatized as an ester (such as an alkyl ester, *e.g.* ethyl ester) or an amide (*e.g.* primary carboxamide, an N-alkyl secondary carboxamide, or an N,N-dialkylcarboxamide). The C-terminal carboxyl group and/or the N-terminal amino group may also be in the form of a salt.

### DETAILED DESCRIPTION OF THE INVENTION

The present invention focuses on the identification of targets for drug discovery for use in the treatment and prevention of viral diseases associated with HCV infection.

Experiments performed in accordance with the present invention using a modified version of the Sos-recruitment system (SRS) essentially described in US patent 5,776,689, which hitherto has not been considered in context with viral/human protein interaction, surprisingly revealed novel human host proteins as specific targets of one of the HCV proteins, *i.e.* NS3. Thus, the new proteins GBP2 (SEQ ID NO. 1) and March7 (SEQ ID NO. 2) involved in HCV infection could be identified, thereby providing novel targets for drug development.

The first protein is the Interferone-induced guanylate binding protein-2 (GBP2), (Synonyms: Guanine nucleotide-binding protein). GTP-binding protein has the accession number: NM_004120. Guanylate-binding proteins (GBPs) define a group of proteins that are synthesized after activation of the cell by interferons (Cheng et al., Mol Cell Biol. Sep; 11(9):4717-25, (1991), Cheng YS, et al. J Biol Chem. Jun 25; 258(12):7746-50, (1983)). The biochemical properties of GBPs are clearly different from those of Ras-like and heterotrimeric GTP-binding proteins. They bind guanine nucleotides with low affinity (micromolar range, Praefcke et al., J Mol Biol (292), 321-32, 1999), are stable in their absence and have a high turnover of GTPase activity (Prakash et al., NATURE (403): 567-71, 2000).The nucleotide binding site give a unique appearance not found in the canonical GTP-binding proteins. For human GBP-1 it was shown to contribute to antiviral activities of IFNs (Anderson S.L. et al., Virology (256), 8-14 (1999)). No protein complexes between NS3 and GBP2 were characterized so far.

The second protein is the membrane-associated Ring-finger 7 (MARCH7), (Previous name: axotrophin, synonyms are membrane-associated RING-CH protein VII). The accession number is NM_022826. MARCH 7 belongs to the family of membrane associated Ring-CH proteins with the configuration C3HC4. The MARCH7 family comprises 11 cellular gene products. Initially this protein class was identified in viruses. Viral transmembrane ring finger ligases, were shown to modulate immune responses by the degradation of membrane proteins, *e.g.* MHC-I degradation was observed by the proteasome in cells expressing the herpesviral protein MK3 (Boname et al. Immunity. Oct;15:627-36, 2001). Their cellular orthologues were shown to have a role in intracellular trafficking (Nathan et al., Traffic 2008; 9: 1130-1145, 2008). However, until now no protein complexes with viral proteins were observed.

Thus, in one aspect, the present invention relates to a composition comprising the viral NS3 protein from HCV or peptide fragments thereof having a length of at least 6 amino acid residues and the MARCH7 protein according to SEQ ID NO. 2 or as encoded by SEQ ID NO. 4 or peptide fragments thereof having a length of at least 6 amino acid residues, wherein the protein or peptide fragments of NS3 are capable of binding said MARCH7 protein.

The fragments may be 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids long or longer.

The following nucleic acid and protein sequences are disclosed herein.

| | |
|---|---|
| Full length GBP2 protein sequence (SEQ ID NO. 1) | |
| | |
| Full length MARCH7 protein sequence (SEQ ID NO. 2) | |
| Full length GBP2 nucleic acid sequence (SEQ ID NO. 3) | |
| | |
| Full length MARCH7 nucleic acid sequence (SEQ ID NO. 4)-The sequence comprises two varying positions which however do not alter its function (underlined) | |
| | |
| Homo sapiens membrane-associated ring finger (C3HC4) 7 (MARCH7), (not only | |
| coding part) mRNA. NM_022826.2 (SEQ ID NO. 14) | |
| | |
| Full length NS3 protein sequence (SEQ ID NO. 5); The sequence comprises varying positions | |
| (underlined as follows: | |
| A to T | |
| G to E | |
| G to R | |
| S to N | |
| T to M) which however do not alter its function; | |
| NS3 protein full length (Hepatitis C virus) gb ACA48642.1 (SEQ ID NO. 15) | |
| Full length NS3 nucleic acid sequence (SEQ ID NO. 6)The sequence comprises some varying positions as compared to AF011752 (Hepatitis C virus strain H77 pCV-H11 polyprotein | |
| gene, complete cds; underlined) which however do not alter its function: | |
| A to G | |
| C to T | |
| T to C | |
| G to A | |
| G to A | |
| G to A | |
| C to T | |
| C to T | |
| T to C | |
| G to A | |
| G to A | |
| C to T | |
| Full length NS3 nucleic acid sequence Genbank AF011752.1 (SEQ ID NO. 16) | |
| | |
| Linker (SEQ ID NO. 7) | GGGGSGGGGS |
| HCV NS3 Helicase domain containing protein sequence (SEQ ID NO. 8); | |
| the sequence comprises some varying positions when compared to gb ACA48642.1 which however do not alter its function: | |
| G to E | |
| S to N | |
| T to M | |
| HCV NS3 Helicase domain containing protein sequence Genbank ACA48642.1 (SEQ ID NO. 17) | |
| HCV NS3 Helicase domain containing DNA sequence (SEQ ID NO. 9) The following positions vary when compared to Genbank: | |
| Bp 29 G to A | |
| Bp 64 C to T | |
| Bp 126 C to T | |
| Bp 549 T to C | |
| Bp 710 G to A | |
| Bp 1016 C to T | |
| | |
| HCV NS3 Helicase domain containing DNA sequence Genbank AF011752.1 (SEQ ID NO. 18) | |
| | |
| Vif protein sequence (SEQ ID NO. 19) | |
| vif DNA sequence (SEQ ID NO. 20) | |
| NS3 primer 1 (SEQ ID NO. 10) | ATCAACTCCAAGCTTACCATGGCGCCCATCACGGCGTACGC |
| NS3 primer 2 (SEQ ID NO. 11) | GCCCCCGGGAAGCTTTGTGACGACCTCCAGGTCGGCCGA |
| NS3 HCF primer 1 (SEQ ID NO. 12) | AAATATGCGGCCGCCTATGTGACGACCTCCAG |
| NS3 HCF primer 2 (SEQ ID NO. 13) | TTTTCGGACCGGTGGACTTTATCCCTG |

The viral NS3 protein is from HCV.

Preferably, the viral NS3 protein from HCV has a sequence according to SEQ ID NO. 5 or SEQ ID NO. 15 is encoded by a sequence according to SEQ ID NO. 6 or SEQ ID NO. 16.

Sequences SEQ ID NO. 5 and 6 differ from the sequences found in Genbank. However, this does not alter their function.

In one aspect of the invention the two proteins or peptides are in a complex.

In one aspect of the invention the invention relates to an antibody that specifically binds to the complex or the binding domain of the corresponding HCV protein and/or the human protein or peptide, respectively.

The invention relates to a method for screening compounds, capable of modulating complex formation and/or complex stability of viral NS3 from HCV and MARCH7 according to SEQ ID NO. 2 or as encoded by SEQ ID NO. 4 comprising the steps of, (a) subjecting a test compound to (i) the composition according to the invention and/or (ii) the complex according to the invention, (b) monitoring changes in complex formation and/or complex stability; and (c) determining a compound as capable of modulating complex formation and/or stability based on its ability to change complex formation between the proteins of (i) and/or change of stability of (ii) compared to a control.

Also disclosed is a method for screening compounds, capable of modulating complex formation and/or complex stability of NS3 and GBP2 comprising the steps of, (a) subjecting a test compound to a composition comprising a GBP2 protein, or the protein encoded by SEQ ID NO. 1, or fragments thereof, (b) identifying such compounds which are capable of binding the GBP2 protein, or the protein encoded by SEQ ID NO. 1, or fragments thereof, (c) subjecting the identified candidate compound from step (b) to (i) the composition NS3 and GBP2; and/or (ii) the complex of NS3 and GBP2, (b) monitoring changes in complex formation and/or complex stability and (c) determining a compound as capable of modulating complex formation and/or stability based on its ability to change complex formation between the proteins of (i) and/or change of stability of (ii) compared to a control.

The invention also relates to a method for screening compounds, capable of modulating complex formation and/or complex stability of viral NS3 from HCV and MARCH7 according to SEQ ID NO. 2 or as encoded by SEQ ID NO. 4 comprising the steps of, (a) subjecting a test compound to a composition comprising a MARCH7 protein, or the protein encoded by SEQ ID NO. 9, or fragments thereof, (b) identifying such compounds which are capable of binding the MARCH7 protein, or the protein encoded by SEQ ID NO. 9, or fragments thereof, (c) subjecting the identified candidate compound from step (b) to (i) the composition according to the present invention; and/or (ii) the complex of the present invention, (b) monitoring changes in complex formation of the proteins or peptide fragments of the composition according to the present invention and/or complex stability of the complex of the composition according to the present inention and (c) determining a compound as capable of modulating complex formation and/or stability based on its ability to change complex formation between between the proteins of the composition of the present invention and/or change of stability of the complex of the present invention compared to a control.

The invention also relates to a kit or assay system comprising a composition according to the invention and/or an antibody according to the invention.

During initial optimization experiments performed in accordance with the present invention and required for establishing an efficient method, it surprisingly turned out that identification of proteins interacting with NS3 ("candidate proteins" and "host proteins", respectively) was considerably improved in the presence of an additional peptide linker molecule. The use of the linker, having the sequence Gly-Gly-Gly-Gly-Ser-Gly-Gly-Gly-Gly-Ser (SEQ ID NO. 7) and a description of which or similar can be found in for example Evers et al. Biochemistry 45 (2006), 13183 and Maeda et al., BioTechniques 20 (1996), 116, when arranged between the effector molecule and the candidate protein, resulted in the possibility of identifying human proteins being a target of NS3, which hitherto have not been identified using conventional methods.

The protein-protein interaction is indicated by the occurrence or absence of cell growth. Therefore, it is necessary to use cells, the phenotype (cell growth) of which changes upon protein-protein interaction. There are cell lines known in the art revealing a respective phenotype, for example, due to the lack of an endogenously expressed effector which is necessary for activating the Ras signalling pathway, which finally induces cell growth. One such cell line, for example, is a yeast cell line mutated in the cdc25 gene (cdc25-2). Cdc25 is a guanine exchange factor (GEF) that when localized at the plasma membrane, leads to the activation of Ras; see Petitjean et al., Genetics 124 (1990), 797-806. Accordingly, mutations in Cdc25-2 lead to lack of an expressed functional Ras effector, resulting in a growth defect at the restrictive temperature, i.e. 37°C. This defect can be overcome by providing or "recruiting" a respective Ras effector to/at the cell membrane. In this context, since the fusion proteins according to the present invention are designed in that one fusion protein comprises a cell membrane localization domain and the other fusion protein comprises an effector molecule, capable of activating the Ras signalling pathway, upon protein-protein interaction the Ras effector is recruited to the cell membrane thereby activating the Ras signalling pathway and enabling the cells to grow. Thus, since the use of said cells renders the method very easy to determine protein-protein interaction by optically observing the presence or absence of cell growth, in a particularly preferred embodiment of the method of the present invention, the yeast cells are *Saccharomyces cerevisiae* cdc25-2 cells.

As mentioned *supra,* during experiments of the present invention, growth of the cells in which the assay is performed is indicative for the protein-protein interaction. However, as already described, this requires that one fusion protein comprises the cell membrane localization domain and that the other fusion protein comprises an effector molecule. Although, there are several molecules known in the art capable of effecting a respective Ras-activation. The effector molecule is the Son of sevenless (Sos)-protein.

In general, the first and second nucleic acid molecule, encoding the first and second fusion protein, respectively, used in the method of the present invention, is present in an expression vector suitable for the particular cells in which the interaction of the fusion proteins is to occur. Examples of appropriate expression vectors comprise, for example, yeast expression vectors or mammalian expression vectors, depending on the cells in which the method is to be performed. In particular, vectors according to the present invention contain a cloning site such as a multiple cloning site, which permits a convenient means to insert a nucleic acid molecule encoding a target protein. In accordance with the present invention, promoter and nucleic acid molecules are arranged in so called "expression cassettes", wherein the expression cassette of the first fusion protein comprises a promoter operably linked to a nucleic acid encoding the target protein (NS3 or a homolog, derivative or fragment thereof) in frame with the effector protein, and the other expression cassette comprises a promoter operable linked to a nucleic acid encoding the candidate (host) protein (or a homologue, derivative or fragment thereof) in frame with the linker and cell compartment localization signal. In addition, the vectors can contain appropriate transcription or translation start or stop signals or the like. Preferably, the expression cassettes are chimeric expression cassettes including heterologous promoters.

The vector further comprises a nucleic acid molecule encoding the target HCV protein as defined above or a binding fragment thereof.

Although several methods for investigating protein-protein interactions are described in the art, the development of methods for identifying interacting partners of viral proteins and detecting the interaction of a viral protein with a host protein, respectively, under physiological conditions is of major interest.

As already mentioned *supra,* one example of a novel anti-HCV strategy may comprise the supply of the identified proteins or mimetics thereof, or the administration of compounds, capable of either influencing the expression of the respective identified protein or capable of affecting (preventing or destructing) respective complexes between the identified host protein and NS3. However, there may be several modes to modulate either the interaction of NS3 with the identified (human) host protein or the individual protein(s). Thus, these newly identified binding partners of NS3 represent novel targets for therapeutic intervention and pharmacologic modulation, respectively.

Furthermore, in addition to the proteins identified by the method of the present invention as being a target of NS3, the complex between NS3 and host protein may also be used for analytic, therapeutic or diagnostic applications in the field of HCV induced diseases. In particular, complexes between NS3 and host proteins identified according to the method of the present invention, preferably between NS3 and the above-referenced proteins also provide suitable targets for respective analysis or development of novel anti-HCV strategies. However, a complex according to the present invention does not only comprise full length proteins but also fragments of target and host proteins as well as homologues or derivatives thereof. In this context, it will be understood that the complex according to the present invention may comprise any fragment or minimum portion of the target and/or host protein as long as they are capable of forming a respective complex interaction. The protein-protein complexes of the present invention may be used, for example, for diagnosing HCV disease development, in the performance of which they can be incorporated into a protein microchip or microarray.

The invention also relates to the use of nucleic acids encoding the novel targets identified herein for the production of the compositions or the complexes disclosed herein.

Furthermore, a protein may be desirable which generally corresponds to the protein identified by the present invention, but which is not longer capable of forming a complex with NS3. These proteins may be achieved by for example modulating the amino acid sequence of an identified protein, for example, by substitution, deletion or addition, wherein those modifications as well as the method of how to affect them are known to the person skilled in the art. In particular, those proteins may be considered as useful for analytical purposes such as for determining the binding region and amino acids essential for binding to NS3, respectively. However, there are several purposes conceivable for designing and using respective proteins, capable for example on one hand to exert the "usual healthy function" but on the other hand not able to bind to NS3, thereby denying the viral access to the cell and to maintain the usual cellular function. Hence, in another embodiment, the present invention relates to a protein derived from the protein encoded by the nucleic acid molecule identified in accordance with the present invention by way of amino acid substitution, deletion and/or addition which is no longer capable of forming a complex as characterized above.

Even further provided is an antibody that specifically binds to the complex as characterized above or the binding domain of the corresponding HCV protein and the human protein, respectively, or the above referenced protein which is derived from the protein encoded by the nucleic acid molecule of the present invention, but which by way of amino acid sequence alterations is no longer capable of forming a respective complex.

Suitable antibodies useful in accordance with the present invention are preferably monoclonal antibodies, but also synthetic antibodies as well as fragments of antibodies, such as Fab, Fv or scFv fragments etc. Antibodies or fragments thereof can be obtained by using methods which are described, *e.g.,* in Harlow and Lane "Antibodies, A Laboratory Manual", CSH Press, Cold Spring Harbor, 1988 or European patent application EP-A 0 451 216 and references cited therein. The production of chimeric antibodies is described, for example, in international application WO 89/09622, and in particular, methods for the production of humanized antibodies are described in, *e.g.,* EP-A1 0 239 400 and WO 90/07861. Further sources of antibodies to be utilized in accordance with the present invention are so-called xenogeneic antibodies. The general principle for the production of xenogeneic antibodies such as human antibodies in mice is described in, e.g., international applications WO 91/10741, WO 94/02602, WO 96/34096 and WO 96/33735.

One way of how to discover respective compounds that my interact with the complex may comprise exposing the cell in which the interaction is to occur and to be affected, for example, inhibited, NS3 itself or the interacting protein to compounds, *i.e.* test compounds with respect to their capability to exert the above-exemplified effects. In particular, a test compound may be subjected to the cell in which the assay is to be performed prior, during or after complex formation between the HCV target protein or a fragment thereof with its putative interacting partner such as a host protein. Changes upon contact with the compound in complex formation and/or stability can be monitored and the test compound will be selected for its capability of modulating the binding of NS3 to the MARCH7 protein.

The compound's mode of action can be different, i.e. it may interact with NS3 itself or the host protein, preferably identified by the method of the present invention, wherein said interaction may occur at the binding site of the protein, usually responsible for binding to the respective interaction partner, thereby blocking said site. However, the interaction may also occur at a site, usually not directly involved in binding to a potential interacting molecule, thereby for example changing the protein's conformation leading either to disappearance or alteration of the binding site and as a consequence preventing the afore-mentioned interaction. Thus, the test compound may act either as a competitive or allosteric inhibitor. Furthermore, in accordance with the present invention, "modulating" or "affecting" does not mean only to affect the interaction in the course of its formation, but also to act on already formed complexes, i.e. to destruct them. However, as already mentioned above, in case of successful interference with said host protein/NS3 interaction, the virus is obstructed in its access to the host's cell machinery and viral infection and spread, respectively, should be prevented or at least limited to a high extent.

In particular, the methods using the protein complexes according to the present invention can also be used for functional studies and quality control of NS3-related therapeutic polynucleotides, proteins, peptides or antibodies as described in, for example, international application WO 06/111866. Thus, in a particular embodiment, the present disclosure also provides to the above referenced method for analysis of efficiency of a known antiviral drug.

Although there are several ways known in the art to detect complex formation, stability and binding constants of complexes, respectively, in the absence and presence of a potential modulating compound, and methods such as isothermal calorimetry, surface plasmon resonance spectroscopy, fluorescence techniques and NMR-spectroscopy are popular to be used for the respective investigations and may also be used in the present invention, however, one drawback of the above methods is their requirement of an extensive experimental setup and expensive equipment. Therefore, in a preferred embodiment of the present invention, the complex formation and/or stability is tested in a GST-pulldown experiment followed by SDS gel analysis. The performance of SDS-PAGEs is known to the person skilled in the art and well described in the pertinent textbooks such as the current editions of Molecular Cloning: A Laboratory Manual, (Sambrook et al., (1989) Molecular Cloning: A Laboratory Manual, 2nd ed., Cold Spring Harbor Laboratory Press. For testing known pharmaceutical compounds and drugs for their real antiviral effect it is generally referred to the standard textbook *"*In vitro Methods in Pharmaceutical Research", Academic Press, 1997.

According to the method of the present invention a compound or collection of compounds is assessed as to be capable of modulating the formation of a complex when it is hampered and decreased, respectively, in the presence of said compound or collection of compounds compared to a control where no compound is added. The same applies of course when testing for complex stability and binding constant, respectively, where a compound is assessed as to be capable of modulating complex stability when said stability is weakened (decreased) in the presence of said compound compared to a control where no compound is added. In general, according to the present invention, the decrease of complex formation or complex stability compared to performing the method without the test compound is indicative for a putative prospective therapeutic agent (drug). The so identified drug can further be placed into any standard assay to test its effect on the HCV life cycle, i.e. for example viral replication and multiplication, respectively. A drug is then selected that preferably rescues and/or confers resistance to disorders mediated by HCV infection.

From the above, the present disclosure provides two newly identified host proteins binding to NS3 and respective complexes with the HCV protein, i.e. viable targets for screening compounds that interfere with the HCV life cycle and thus ameliorate viral diseases. Compounds in accordance with the present invention can comprise any substance which exhibits the desired effect, i.e. being capable of interfering with the interaction of NS3 with MARCH7 by affecting either NS3, MARCH7 or their respective interaction. Such compounds include but are not limited to peptides, polypeptides, PNAs, peptide mimetics, antibodies, nucleic acid molecules, aptamers or small organic compounds, either naturally occurring or synthetically prepared.

Once a potential compound is identified, additionally chemical analogues can be either selected from a library of chemicals as are commercially available from most large chemical companies including Merck, GlaxoWelcome, Bristol Meyers Squib, Monsanto/Searle, Eli Lilly, Novartis and Pharmacia UpJohn, or alternatively synthesized *de novo.* The synthesis of those potential compounds is known to the person skilled in the art.

Although the compounds which can be identified by the above mentioned method to be capable of affecting the interaction of NS3 with its interacting molecule such as (human) host protein are not limited, however, in a preferred embodiment of the present invention, the compound is selected from the group consisting of antibodies, proteins, peptides and aptamers, and preferably is a peptide. In a further preferred embodiment of the present invention, the peptide is derived from the HCV target protein or from the human protein and preferably consists of about 10 to 20 amino acids.

Suitable peptides may also be obtained by the method for particularly detecting protein-peptide interactions *in vivo* and isolating the respective interaction partner(s) as disclosed by the applicant in European patent application EP 1 895 304 "Means and methods for detecting protein-peptide interactions", and its subsequent international application WO 2008/025564. However, of course also synthetic peptides for example derived from proteins identified according to the method of the present invention are envisaged and can be prepared using the well known techniques of solid phase, liquid phase, or peptide condensation techniques, or any combination thereof. They can include natural and unnatural amino acids. Useful peptides may comprise D-amino acids, a combination of D- and L-amino acids, and various "designer" amino acids (*e.g.* β-methyl amino acids, Ca-methyl amino acids, and Na-methyl amino acids, etc.) to convey special properties. As already mentioned in connection with proteins derived from those identified according to the method of the present invention, but comprising a modified amino acid sequence, also peptides may be used, for the identification of binding regions or minimum required amino acid sequence to form a respective complex.

The present invention also contemplates the validation of compounds or agents which are known to bind to any one of the NS3 interacting proteins, identified according to the method of the present invention, but hitherto have not been considered to be useful in the treatment of viral diseases. Such compounds may be easily retrieved from the literature concerning any one of the NS3-interacting proteins, for example in patent databases such as "espacenet" hosted by the European Patent Office or in databases of public literature, *e.g.* "Medline". In addition, the person skilled in the art may identify compounds to be used in accordance with the present invention by screening so-called "primary databases" such as "Genbank", "EMBL" or "UniprotKB/Swiss-Prot" for nucleotide and protein sequences, respectively, for example by entering the Accession Number or the IUPAC-nomenclature or the name of the protein. The nucleotide and amino acid sequences in the mentioned databases are usually annotated with corresponding citations which in term provide further information with respect to regulation of the corresponding genes and thus guidance for modulating agents to be used in accordance with the present invention. In addition, so called "secondary databases" can be used, for example "PROSITE", "PRINTS", "Pfam", "INTER Pro", "SCOP" or "CATH", being database of protein families and domains, providing fingerprints as classification of sequences, or protein structures. A most suitable web interface allowed to start searching is provided by "Entrez" of NCBI and sequence retrieval system "SRS", respectively. Often a search with keywords in "Google" will already be successful in identifying suitable sources of information.

For the screening method of the present invention the target protein, the host protein or the compound, capable of affecting the interaction may be affixed to a solid surface. One way to perform the method of the present invention can be, for example, to attach the target protein (NS3) to a solid support which is then washed to remove excessive target protein which is not attached. Subsequently, the support is contacted with and accordingly NS3 is exposed to, for example, a labelled candidate protein to be tested for interaction or a test compound. Afterwards, the solid support is washed again to remove the protein or compound not bound to the target protein and the amount remaining with the solid support and thereby identified as to be bound to NS3 can be determined. Alternatively, or in addition, the dissociation constant between the labelled compound and NS3, for example can be determined. Suitable labels for either NS3, the candidate protein or the compound are well known in the art and include enzymes, fluorophores (*e.g.,* fluorescene isothiocyanate (FITC), phycoerythrin (PE), Texas Red (TR), rhodamine, free or chelated lanthanide series salts, especially Eu³⁺, to name a few fluorophores), chromophores, radioisotopes such as ³⁵S, chelating agents, dyes, colloidal gold, latex particles, ligands (*e.g.* biotin), and chemiluminescent agents.

Hence, in a further preferred embodiment of the method of the present invention the HCV target protein, the human protein or the compound to be screened is arranged on a solid support, preferably wherein the solid support is an array or chip, such as a micro chip or a microarray. In this context, the present invention naturally also relates to a chip or array for use in the methods of the present invention.

Methods for attaching for example proteins to a solid support are well known in the art and include for example linking a tag to a target protein. In accordance with the method of the present invention the tag is, for example, glutathione-S-transferase (GST) and the target protein is NS3, preferably, wherein NS3 is expressed as the respective NS3-GST fusion protein. Furthermore, a respective solid support and matrix, respectively is provided, such as glutathione(GSH)-agarose beads. After contacting the GST-tagged NS3 to the solid support and subsequent washing to remove unreacted species, NS3 is bound to the support via the glutathione-GST-interaction. There are several further ways for attaching the protein to a solid support such as linking biotin to NS3 and linking avidin to the solid support. It is within the general knowledge of the person skilled in the art that the choice of the respective suited way of how to attach the protein to the solid support may depend on individual characteristics of the assay to be performed and the experimental setup, respectively.

However, in a particular preferred embodiment of the method of the present invention for screening compounds, capable of modulating complex formation and/or complex stability, the HCV target protein is a fusion protein comprising glutathione-S transferase and preferably, the human protein is labelled with S³⁵-methionine.

In an even more preferred embodiment of the present invention, the complex formation results from protein-protein interaction in a cell-based two- or three-hybrid system.

Although the above assay has been exemplarily described for the target protein (NS3) to be attached to the solid support, the person skilled in the art knows that this assay of course can be performed *vice versa*, i.e. by attaching for example the candidate protein to the solid support.

When performing screening methods, it is often intended to screen a variety of compounds in one experiment. Therefore, the methods of the present invention generally can be designed in a format for use in conventional laboratory or adapted for high throughput screening. In this context, the term "high throughput screening" (HTS) refers to an assay design that allows easy analysis of multiple samples simultaneously, and capacity for robotic manipulation. Another desired feature of high throughput assays is an assay design that is optimized to reduce reagent usage, or minimizes the number of manipulations in order to achieve the analysis desired.

Of course in a further embodiment, the present invention relates to a compound which could have been identified or was obtainable by the method of the present invention, preferably wherein said compound hitherto has not been disclosed in the prior art as a drug for the treatment of a viral disease, in particular a lentiviral disease, such as AIDS.

Furthermore, the present invention relates to a pharmaceutical composition comprising an antibody or a compound according to the present invention and optionally a pharmaceutically acceptable carrier. The pharmaceutical composition of the present invention can be formulated according to methods well known in the art; see for example Remington: The Science and Practice of Pharmacy (2000) by the University of Sciences in Philadelphia, ISBN 0-683-306472. Suitable pharmaceutically acceptable carriers can be taken from corresponding literature and include, for example, phosphate-buffered saline solutions, water, emulsions, such as oil/water emulsions, various types of wetting agents, sterile solutions etc. The pharmaceutical compositions can be administered to the subject at a suitable dose, wherein the suitable compositions may be effected by different ways, i.e., by suitable administration routes some of which are described in the respective literature and include, for example, oral, intranasal, rectal, topical, intraperitoneal, intravenous, intramuscular, subcutaneous, subdermal, transdermal, intrathecal, and intracranial administration. Aerosol formulations such as nasal spray formulations include for example purified aqueous or other solutions of the active agent with preservative agents and isotonic agents. Such formulations are preferably adjusted to a pH and isotonic state compatible with the nasal mucous membrane. Formulations for rectal or vaginal administration may be presented as a suppository with a suitable carrier. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

The dosage regimen will be determined by the attending physician and clinical factors. As is well known in the medical arts, dosages for any one patient depends on many factors, including the patient's size, body surface area, age, the particular compound to be administered, sex, time and route of administration, general health, and other drugs being administered concurrently. Preparations for parenteral administration may include sterile aqueous or non-aqueous solutions, suspensions, and emulsions. Examples of non-aqueous solvents comprise for example propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose) and the like. Preservatives and other additives may also be present such as, for example, antimicrobials, antioxidants, chelating agents, and inert gases. Furthermore, the pharmaceutical composition may also be formulated as a vaccine, for example, if the pharmaceutical composition of the invention comprises an anti-HCV antibody for passive immunization.

In addition, co-administration or sequential administration of other agents may be desirable. A therapeutically effective dose or amount refers to that amount of the active ingredient sufficient to ameliorate the symptoms or condition. Therapeutic efficacy and toxicity of such compounds can be determined by standard pharmaceutical procedures in cell cultures or experimental animals, *e.g.,* ED50 (the dose therapeutically effective in 50% of the population) and LD50 (the dose lethal to 50% of the population). The dose ratio between therapeutic and toxic effects is the therapeutic index and it can be expressed as the ratio LD50/ED50. As mentioned already *supra,* dosage of the agent will depend on the treatment, route of administration, the nature of the therapeutics, sensitivity of the patient to the therapeutics, etc. Utilizing LD50 animal data, and other information, a clinician can determine the maximum safe dose for an individual. The composition can be administered to the subject in a series of more than one administration. For therapeutic compositions, regular periodic administration will sometimes be required or may be desirable. Therapeutic regimens will vary with the agent, e.g. a small organic compound may be taken for extended periods of time on a daily or semi-daily basis, while more selective agents such as peptide mimetics or antibodies may be administered for more defined time courses, e.g. one, two, three ore more days; one or more weeks; one or more months etc.; taken daily, semi-daily, semi-weekly, weekly, etc.

Further to the detection and identification of proteins forming a complex with NS3 as well as compounds, capable of affecting said complex formation and/or -stability, one major interest relates to the "nature" of the complex, in particular the complex according to the present invention, i.e. formed between viral- and host factor. Since, in particular when considering therapeutic approaches, the complex itself may be considered as a drug target, knowledge of the characteristics of the respective complex is necessary for the development of therapeutic agents and therapies, respectively. Typical characteristics of a complex comprise physical or structural characteristics such as stability, type, size (multimeric or monomeric complex), shape and three dimensional structure, but also biological properties such as the amino acid composition of the binding region or the type of interactions in the binding region as well as the impact of complex formation on, for example, generating further binding sites the individual complex partners would not have disposed. Generation of those alternate binding sites may be due to, for example, conformational change of at least one complex partner during complex formation or lack of previous binding sites because of either occupying said site by the complex partner or by conformational rearrangement, thereby leading to the disappearance of former binding sites. Furthermore, knowledge of the lifetime of a complex may be important, which depends on various parameters known in the art, such as salt concentration, pH value and the like. Additionally, chemical, magnetic or electrostatic properties may be of interest, for example, the type of interaction by which the complex is held together such as hydrogen bonds as well as surface charge of the complex.

As already mentioned, the knowledge of the above exemplarily enumerated characteristics is important for prediction of, for example, complex behavior or potential binding regions (in particular for agents, capable of modulating formation and/or stability of the complex, and drug targeting). Hence, the knowledge of complex properties provides necessary information, when considering how to affect the respective complex for various purposes such as to destroy it or prevent its formation. As the person skilled in the art will know, there are several ways of how to prepare, detect, and analyze a respective complex. Those methods may include procedures of formation of protein complexes using *in vitro* and *in vivo* systems, which can be adapted to the present invention. For instance, methods may comprise synthesis and isolation of the first protein NS3 and the second protein selected from the group of host cell proteins and formation of protein complexes using well known *in vitro* methods. Alternatively, the protein complexes can also be isolated or purified from tissues or cells or produced by recombinant expression of their protein members. Which method is to be used will depend on the type of complex, purpose of investigation as well as individual experimental parameters.

However, in one particular embodiment, the present invention provides a method of preparing, detecting and analyzing the complex of the present invention and defined above, comprising the steps of, (a) synthesizing and/or isolating the HCV protein (i.e. NS3), (b) synthesizing and/or isolating the human protein (i.e. MARCH7), (c) contacting proteins of (a) and (b), (d) monitoring complex formation, and optionally, (e) determining complex characteristics (stability or kinetics) by known *in vitro* methods.

In yet another embodiment the present invention relates to a diagnostic composition comprising the nucleic acid, the vector, the recombinant host cell, the protein, the complex, the antibody, the compound and/or the chip or array of the present invention.

In a further embodiment, the present invention also relates to a kit or assay system for use in the methods of the present invention, said kit or assay system comprising a component of the present invention as described *supra,* and/or reagents, automation tools, storage devices, liquid handling robots, monitoring arrangements or the like. In particular, the present invention relates to a kit for use in any one of the methods as described above, i.e. for identifying, cloning, preparing, screening, monitoring, determining, testing, analyzing and/or using the nucleic acid molecules, proteins, compounds, complexes and complex formation, respectively, or compositions of the present invention. Therefore, such a kit preferably comprises the essential components such as the target protein (NS3), the candidate (host) protein (MARCH7) or fragments thereof, or recombinant nucleic acid molecules encoding the respective proteins (NS3, MARCH7 or fragment), more preferably in the form of a corresponding first and second expressible nucleic acid molecule. Even more preferably, the expressible nucleic acid molecules are present in an expression vector suitable for the particular cells in which the interaction assay is performed. Appropriate expression vectors can be, for example, yeast expression vectors or mammalian cell expression vectors, depending on the cells in which the method is to be performed.

If desired, the kit further contains reagents, for example, that result in optimal transfection efficiency of the nucleic acids for the particular host cell type. In addition, appropriate host cells can be included in a kit, although such cells generally are available or can be selected for a particular embodiment of the method. Preferably, the kit of the present invention contains reagents such as those described hereinbefore useful for conducting any of the above-described methods of the present invention, comprising for example selectable markers, medium or media components, reference samples, microarrays, culture vessels, vectors, proteins, peptides, and maybe a suitable detection means, spectroscopic devices and/or monitoring systems, capable of monitoring complex formation, i.e. decrease or increase of complex formation of (optionally tagged) NS3 and its interacting molecule(s) such as (optionally tagged) host protein(s). Furthermore, an increased or decreased binding capacity compared to a control may be detected by, for example, labels comprising fluorescent label, phosphorescent label, radioactive label, which are known to those skilled in the art. Optionally, the kit further comprises instructions on how to perform any method of the present invention, preferably the use of the kit in the methods concerning the identification and/or cloning of nucleic acid molecules encoding interacting molecules of NS3 or validation or assessment of potential drugs, agents, compositions or compounds influencing (inhibiting or enhancing) said interaction. These instructions can be provided to detail the use of the components of the kit, such as written instructions, video presentations, or instructions in a format that can be opened on a computer, e.g. a diskette or CD-ROM disk.

Furthermore, such a kit would typically comprise a compartmentalized carrier suitable to hold in close confinement at least one container and the compounds of the kit may be sterile, where appropriate. The kit may further include a transfer means, such as pipettes, for transferring any fluent component. The kit of the present invention is preferably suitable for commercial manufacture and scale and can still further include appropriate standards, positive and negative controls.

It will be apparent that the methods and components of the present invention, the proteins obtained thereby, as well as the uses as substantially described herein or illustrated in the description and the examples, are also subject of the present disclosure. In this respect, it is also understood that the embodiments as described in any one of the examples, can be independently used and combined with any one of the embodiments described hereinbefore and claimed in the appended claims set. Thus, these and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example, the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The disclosure also relates to a diagnostic assay for the detection of HCV, wherein a complex between any of the target proteins or peptides disclosed herein and NS3 is detected. Such an assay may make use of blood or any other tissue. Hence, the disclosure also relates to a diagnostic assay for HCV detection wherein any of the target proteins herein is used.

The peptides claimed herein may be generated as follows. The nucleic sequence encoding the MARCH7 protein is digested either enzymatically or mechanically sheared in such a way that small fragments are generated. The fragments shall have the desired length. The fragments are cloned into a vector for expression of the encoded peptide. Various peptides are tested for binding to NS3. A binding region may be determined. Also, peptides of a give length may be synthesized chemically and binding to NS3 may be tested.

### EXAMPLES

The examples which follow further illustrate the invention, but should not be construed to limit the scope of the invention in any way. Detailed descriptions of conventional methods such as those employed herein can be found in the cited literature; see also "The Merck Manual Diagnosis and Therapy" Seventeenth Ed. Ed. By Beers and Berkow (Merck&Co., Inc. 2003)

The practice of the present invention will employ, unless otherwise indicated, conventional techniques of cell biology, microbiology, recombinant DNA, and immunology, which are within the skill of the art.

### Example 1: Identification of novel NS3 interacting proteins

The example demonstrates the identification of protein complexes of a recombinant HCV NS3 (SEQ ID NO. 5) respectively HCV NS3 HCF (SEQ ID NO. 8) with novel human polypeptides encoded by a human hepatocyte cDNA library (CytoTrap® XR Human Liver cDNA Library, Stratagene).

### Bait construction

The HCV NS3 FL sequence (aa 1-631) and HCV NS3 HCF sequence (aa 167-631) was cloned into an appropriate vector for expression of heterologous proteins in yeast. NS3 FL was cloned in pADNSOS as N-terminal fusion protein, NS3 HCF was cloned in pADH-SOS-2xSpc as c-terminal fusion protein with Sos including a small spacer (SEQ ID 5). Both vectors were modified from pSOS vector (Sikorski and Hieter, Genetics 122 (1989), 19-27). NS3 cDNA was amplified by polymerase chain reaction (PCR) with the following primers:
5'-ATCAACTCCAAGCTTACCATGGCGCCCATCACGGCGTACGC-3' SEQ ID NO. 10
5'-GCCCCCGGGAAGCTTTGTGACGACCTCCAGGTCGGCCGA-3' SEQ ID NO. 11

The PCR fragment was cloned with in fusion PCR cloning kit into pADNSOS vector linearized at the HindIII restriction site (In-Fusion™ PCR Cloning kit, clontech). The NS3 HCF fragment was amplified by PCR reaction with Primer
5'-AAATATGCGGCCGCCTATGTGACGACCTCCAG-3' SEQ ID NO. 8
5'-TTTTCGGACCGGTGGACTTTATCCCTG-3' SEQ ID NO. 9

Following restriction digest with *Rsr*II *Not*I PCR the product was cloned into the pADH-SOS-2xSpc vector.

### Library amplification

Library transformation was performed in *E. coli* (XL10-Gold® Ultracompetent Cells, Stratagene) according to the instructions of the manufacturer. For the library amplification 1 x 10⁷ individual clones were needed to be sure that most cDNAs are represented in the amplified cDNA library. Therefore 160 mm Petri dishes were used for library amplification. LB chloramphenicol agar was generated by supplementing LB agar with 1 ml of filter sterilized chloramphenicol in a concentration of 30µg ml⁻¹. On every Petri dish about 5 x 10⁴ colony forming units (cfu) can grow. The titer of the purchased hepatocyte cDNA library *E. coli* strain was 3,8 x 10⁵ *E. coli* per µl (manufactures information: 4,7x10⁶). 120µl of the library were mixed with 18ml LB chloramohenicol and spread with autoclaved glass beads (2.85 - 3.3 mm, Roth) on 120 plates, 150µl of the suspension on each plate. After overnight incubation at 37°C, the *E.coli* colonies were counted on a dilution plate and scraped off the plates with a rubber police man. Altogether 21,6 Mio clones were generated. The *E. coli* of 10 plates were transferred into a 50 ml centrifuge tube and carefully resuspended in 50 ml LB-chloramphenicol medium. The content of five such centrifuge tubes was then transferred into a sterile 500 ml centrifuge beaker and centrifuged for 15 min (4°C, 6000 rpm (JA-10)). The cell pellets were resuspended in one cell pellet volume of STE buffer/10% glycerol (STE buffer. 0.1 M NaCl, 10mM Tris pH 8,0, 10% glycerol) and 5 g pellet suspension were frozen in cryotubes at -80°C. 2,5 g cells were thawn for plasmid isolation (Kit AX2000 (Macherey-Nagel) was used).

### Yeast transformation

The transformation of cdc25-2 yeast cells (MATα, ura3, lys 2, Leu2, trpl cdc25-2, his3Δ200, ade 100, GAL+) was performed according to a modified protocol for a high efficient transformation method of Gietz and Schiestl (1989). For preparation of competent yeast cells, 10 ml YEPD medium (1% Bacto yeast extract, 2% tryptone, 2% Glucose) was inoculated with a single colony of cdc25-2 yeast cells and incubated on a rotary shaker at approximately 200 rpm over night at 24°C. An appropriate volume of the overnight culture was used to inoculate 50 ml YEPD medium to get an OD₆₀₀ of 0.2 per ml. After incubation at 24°C for two generations (until OD₆₀₀~0.8) the culture was sedimented (1000 x g, 5 min, room temperature), the pellet was washed with sterile ddH₂O and centrifuged as described before. Afterwards the pellet was resuspended in 10 ml LiSORB (100mM Lithium acetate, 1M D-sorbitol, in 10mM Tris, 1mM EDTA; pH 7.5; filtersterilized) and again sedimented. The pellet was resuspended in 50 µl LiSORB per transformation (maximal 500 µl). The cells were directly used for transformation. For preparation of the carrier DNA-mix 10 µl fragmented Carrier-DNA (salmon sperm DNA, 10 mg ml⁻¹ in TE buffer) per transformation was boiled at 95°C for 5 min. The carrier DNA was then mixed with 40 µl LiSORB per transformation and placed on ice for cooling to room temperature.

For the actual transformation, 0.5-1 µg plasmid-DNA, 50 µl carrier DNA-mix and 50 µl competent yeast cells were mixed. The mixture was incubated for 30 min at 24°C with shaking. 900 µl LiPEG (100 mM Lithium acetate (pH 7.5), 40 % (w/v) PEG4000, in 10mM Tris, 1 mM EDTA (pH 7.5), filter-sterilized) were added to each sample and again incubated at 24°C with shaking for 30 min. 100 µl DMSO were added and the cells were heat shocked for 7 min at 42°C without shaking. The cells were sedimented (1 min, 1000 g) and resuspended in 100 µl 1M sorbitol (filter sterilized). 50 µl of the suspension was inoculated on an appropriate selection plate and incubated at 24°C. Colonies appeared after 4-6 days.

### Library screening

The hepatocyte library was transformed by a modified version of large scale transformation as described by Gietz and Schiestl (2007) into cdc25-2 yeast strain. 10 clones of cdc25-2 were scrapped off agar plates to inoculate 20ml YEPD in a 100ml Erlenmeyer flask and incubated on a rotary shaker at approximately 200 rpm for 7 hours at 24°C. After measuring the optical density at OD₆₀₀, cells were diluted 1/100 in 200ml YEPD and incubated on a rotary shaker at approximately 200 rpm overnight. Next morning the OD₆₀₀ of a 1/10 dilution was determined. Therefore, 100µl cell suspension was pipetted in 900µl water and mixed. 2x10⁸ cells were transferred to inoculate 800ml 2xYEPD (the final cell titer OD600 was 0,25 per ml). 2xYEPD medium was used with the composition of 2% Bacto yeast extract (w/v); 4% Bacto peptone (w/v) and 4% (w/v) glucose. Cells were incubated at 24°C and 200 rpm until they have undergone two divisions. After incubation, cells were sedimented (1000g, 5min) and 50ml cells were washed with 10ml cold ddH₂0. Cells were sedimented again and washed with 10ml cold LiSorb (100mM Lithium acetate, 1M D-sorbitol, in 10 mM Tris, 1 mM EDTA; pH 7.5; filter sterilized). Incubation for making cells competent was at 24°C 15 min was in LiSorb under shaking at 200 rpm. Cells were pelleted at 1000 x g and resuspended in 600µl LiSorb. Cells were kept on ice until preparation of carrier DNA was completed.

The first step for the preparation of the carrier DNA (Stratagene 10mg/ml in10mM Tris-HCl pH 8.0, 1mM EDTA) was melting at RT. Denaturation was done by heating of 400 µl at 95°C for 5min. For the actual transformation, 2 µg hepatocyte library DNA were cotransformed with 500 ng bait plasmid-DNA. 50 µl carrier DNA-mix and 50 µl competent yeast cells were mixed with library- and bait DNA. The mixture was incubated for 30 min at 24°C under shaking at 700 rpm. 100 µl of DMSO were added and vortexed for 5 sec. Heat shock was done at 42°C for 7min (without shaking). Cells were pelleted after centrifugation at 1000 x g for 2 min at RT. Cells were resuspended in 300 µl sorbitol and plated on 160 mm -Leu-Ura glucose plates (Synthetic drop out medium with 2% glucose final and 18 g Agar/L). Incubation of plates at 24°C for 50-60h resulted in approximately 50000 transformants per plate.

In summary, 70 µg hepatocyte DNA were cotransformed with 17,5 µg NS3 FL bait (pADNSOS NS3FL) or NS3 HCF (pADH-Sos-2xSpc-NS3 HCF) respectively. The number of clones generated with NS3FL was 2x10⁶ with NS3HCF bait was 4,5x10⁵. As negative control for further screening steps 500 ng empty bait construct (pADNSOS, pADH-SOS-2xSpc) was cotransformed with 2µg hepatocyte library DNA. As positive control 200 ng plasmids expressing interacting proteins were transformed

For selection of clones expressing NS3FL or NS3 HCF interacting proteins, transformants were replica plated with membrane filters (Filter Nytran N45) onto-Leu-Ura+galactose plates (Synthetic drop out medium with 3% galactose, 2% raffinose final and 18g Agar/L). Plates were grown at 37°C for 4-10 days. Appearing colonies were picked, resuspended in 150 µl -Leu-Ura+raffinose medium (Synthetic drop out medium with 2% glucose) and incubated over night at RT and 800rpm.

To test whether the growth of putative interaction candidates at 37°C is dependent on prey expression, clones were plated onto -Leu-Ura+galactose plates as well as on - Leu-Ura+glucose plates following incubation at 37°C for 5-7 days. The expression of NS3-interacting fusion proteins is under control of the GAL1 inducible promoter of the library plasmid. Therefore, expression was induced by the addition of galactose to the medium (3 % galactose and 2 % raffinose) and repressed by the addition of glucose (2 % glucose).

Yeast cells expressing NS3 FL or NS3 HCF-interacting proteins of the hepatocyte library are growing only on galactose plates (3 % galactose, 2 % raffinose) but do not grow on glucose plates; see Fig. 1 (clones which comply those criteria are marked by circles).

### Isolation of plasmids comprising an encoded NS3FL or NS3HCF interacting protein

Plasmids were isolated according to a protocol of Michael Jones using the QIAprep® Spin Miniprep Kit (www.qiagen.com/literature/protocols/pdf/PR04.pdf). A single yeast colony was inoculated into 5 ml -Ura+glucose medium and grown at 24°C for 24 h with shaking. Cells were harvested by centrifugation for 5 min at 5000 x g and resuspended in 250 µl Buffer P1 (Qiagen) containing 0.1 mg/ml RNase A (Qiagen). The cell suspension was transferred to a fresh 1.5 ml microcentrifuge tube. For disruption, 50-100 µl acid-washed glass beads (Sigma) were added and the suspension was vortexed for 5 min. After settling of the beads, the supernatant was transferred to a fresh 1.5 ml microcentrifuge tube. Then 250 µl lysis buffer P2 (Qiagen) was added to the tube, the tube was inverted 4-6 times and incubated for 5 min at room temperature. After incubation, 350 µl neutralization buffer N3 (Qiagen) were added to the tube and the tube was inverted immediately but gently 4-6 times. The lysate was centrifuged at 15000 x g for 10 min. Thereafter, the cleared lysate was transferred to QIAprep Spin Column and centrifuged for 1 min at 15000 x g. The column was washed by adding 0.75 ml buffer PE (Qiagen) and centrifugation for 1 min at 15000 x g. Elution of the DNA was performed by adding 25 µl EB (Qiagen) to the center of the QIAprep Spin Column. After 1 min incubation time, the eluate was obtained by centrifugation for 1 min at 15000 x g. Subsequent transformation into CaCl₂ competent E. coli was performed with 10 µl eluate. The bacteria were plated on LB-plates containing 50 µg/ml chloramphenicol. Prey plasmids were isolated from E.coli and analyzed by *Eco*RI/*Xho*I restriction digest which releases the insert

### EXAMPLE 2: DETECTION OF NS3 PROTEIN COMPLEXES IN YEAST CELLS

This example demonstrates that the invention is also useful for the detection of specific protein complexes in yeast by expressing a NS3 fusion protein and fusion proteins of the identified host factors. For the identification of the "positive interactors" identified in Example 1, the DNA of their corresponding library sequence was isolated from the cells. Further to the identification of novel NS3 interacting proteins, this interaction was tested for specificity, i.e. for the dependence of the protein-protein interaction on the expression of the first fusion protein (bait), i.e. the target protein being NS3 FL or NS3 HCF, respectively, comprising the first fusion protein, i.e. NS3 as target protein. Therefore, isolated library plasmids of NS3 interacting proteins were not only introduced into the yeast strain containing the vector pADNSOS-NS3FL or pADH-Sos-2xSpc-NS3 HCF comprising the encoded first fusion protein, i.e. NS3 as target protein, but also additionally introduced into the cdc25-2 yeast strain containing a heterologous bait vector pADH-Sos-2xSpc-vif as control comprising a fusion protein providing Vif as a "negative control" target protein. As described above, cells were plated onto YNB-Leu-Ura, and glucose plates and incubated at 24°C. Clones of each transformation assay were suspended in liquid medium and transferred onto one set of galactose- and two sets of glucose plates (each -Ura-Leu), respectively, and incubated at 37°C. On galactose plates the dependency of the interaction is tested on the expression of the prey protein, which is under the control of a galactose inducible promoter. The glucose plates, incubated at 37°C, were used to test for potential growth of revertants. The set of glucose plates, incubated at 24°C, was used as general growth control. The control vector (providing the "negative control" target protein) at the restrictive temperature of 37°C indicates the binding specificity of the identified novel host cellular interactors to the pADH-Sos-2xSpc bait construct, since there was no growth of cells containing the identified novel host factors in combination with the non-relevant pADH-Sos-2xSpc-cJun construct; see Fig. 2. After demonstrating the specificity of the interacting host proteins for binding to Vif, the DNA of their corresponding library sequence was sequenced.

## Claims

1. Composition comprising the viral NS3 protein from HCV or peptide fragments thereof having a length of at least 6 amino acid residues and the MARCH7 protein according to SEQ ID NO. 2 or as encoded by SEQ ID NO. 4 or peptide fragments thereof having a length of at least 6 amino acid residues, wherein the protein or peptide fragments of NS3 are capable of binding said MARCH7 protein.

2. Composition according to claim 1, wherein the viral NS3 protein from HCV has a sequence according to SEQ ID NO. 5 or SEQ ID NO. 15 or is encoded by a sequence according to SEQ ID NO. 6 or SEQ ID NO. 16.

3. Composition according to claims 1 or 2, wherein the two proteins or peptides are in a complex.

4. A method for screening compounds, capable of modulating complex formation and/or complex stability of viral NS3 from HCV and MARCH7 according to SEQ ID NO. 2 or as encoded by SEQ ID NO. 4 comprising the steps of:
(a) subjecting a test compound to the composition of any of the claims 1 to 3;
(b) monitoring changes in complex formation of the proteins or peptide fragments of the composition of claims 1 or 2 and/or complex stability of the complex of the composition of claim 3; and
(c) determining a compound as capable of modulating complex formation and/or stability based on its ability to change complex formation between the proteins of the composition of claims 1 or 2 and/or change of stability of the complex of claim 3 compared to a control.

5. A method for screening compounds, capable of modulating complex formation and/or complex stability of viral NS3 from HCV and MARCH7 according to SEQ ID NO. 2 or as encoded by SEQ ID NO. 4 according to claim 4, wherein the test compound is identified as a candidate test compound before step (a) by subjecting a test compound to a composition comprising an MARCH7 protein, or the protein encoded by SEQ ID NO. 9, or fragments thereof; and
identifying such compounds which are capable of binding the MARCH7, or the protein encoded by SEQ ID NO. 9, or fragments thereof.

6. A method of preparing, detecting and analyzing the complex between viral NS3 from HCV and MARCH7 according to SEQ ID NO. 2 or as encoded by SEQ ID NO. 4, comprising the steps of:
(a) synthesizing and/or isolating the viral NS3 protein of HCV;
(b) synthesizing and/or isolating the human MARCH7 protein according to SEQ ID NO. 2 or as encoded by SEQ ID NO. 4;
(c) contacting proteins of (a) and (b);
(d) monitoring complex formation; and optionally;
(e) determining complex stability or kinetics by known *in vitro* methods.

7. A kit or assay system comprising a composition according to any of the claims 1 to 3.

## Patentansprüche

1. Zusammensetzung, umfassend das virale NS3-Protein von HCV oder Peptidfragmente davon mit mindestens einer Länge von 6 Aminosäureresten und das MARCH7-Protein gemäß SEQ ID NO:2 oder wie durch SEQ ID NO:4 codiert oder Peptidfragmente davon mit mindestens einer Länge von 6 Aminosäureresten, wobei das Protein oder die Peptidfragmente von NS3 in der Lage sind, das MARCH7-Protein zu binden.

2. Zusammensetzung nach Anspruch 1, wobei das virale NS3-Protein von HCV eine Sequenz gemäß SEQ ID NO:5 oder SEQ ID NO:15 aufweist oder durch eine Sequenz gemäß SEQ ID NO:6 oder SEQ ID NO:16 codiert wird.

3. Zusammensetzung nach Anspruch 1 oder 2, wobei die beiden Proteine oder Peptide in einem Komplex sind.

4. Verfahren zum Screenen von Verbindungen, die in der Lage sind, die Komplexbildung und/oder Komplexstabilität von viralem NS3 von HCV und MARCH7 gemäß SEQ ID NO:2 oder wie durch SEQ ID NO:4 codiert zu modulieren, umfassend die Schritte:
(a) Aussetzen einer Testverbindung gegenüber der Zusammensetzung nach einem der Ansprüche 1 bis 3;
(b) Überwachen der Veränderungen in der Komplexbildung der Proteine oder Peptidfragmente der Zusammensetzung nach Anspruch 1 oder 2 und/oder in der Komplexstabilität des Komplexes der Zusammensetzung nach Anspruch 3; und
(c) Bestimmen, dass eine Verbindung in der Lage ist, die Komplexbildung und/oder Komplexstabilität zu modulieren, auf der Grundlage ihrer Fähigkeit, die Komplexbildung zwischen den Proteinen der Zusammensetzung nach Anspruch 1 oder 2 und/oder die Komplexstabilität des Komplexes nach Anspruch 3 verglichen mit einer Kontrolle zu verändern.

5. Verfahren nach Anspruch 4 zum Screenen von Verbindungen, die in der Lage sind, die Komplexbildung und/oder Komplexstabilität von viralem NS3 von HCV und MARCH7, gemäß SEQ ID NO:2 oder wie durch SEQ ID NO:4 codiert, zu modulieren, wobei die Testverbindung als eine Kandidatentestverbindung vor Schritt (a) identifiziert wird durch Aussetzen einer Testverbindung einer Zusammensetzung, die ein MARCH7-Protein oder das von SEQ ID NO:9 codierte Protein oder Fragmente davon umfasst; und
Identifizieren solcher Verbindungen, die in der Lage sind, MARCH7 oder das von SEQ ID NO:9 codierte Protein oder Fragmente davon zu binden.

6. Verfahren zum Herstellen, Nachweisen und Untersuchen des Komplexes aus viralem NS3 von HCV und MARCH7 gemäß SEQ ID NO:2 oder wie durch SEQ ID NO:4 codiert, umfassend die Schritte:
(a) Synthetisieren und/oder Isolieren des viralen NS3-Proteins von HCV;
(b) Synthetisieren und/oder Isolieren des humanen MARCH7-Proteins gemäß SEQ ID NO:2 oder wie durch SEQ ID NO:4 codiert;
(c) Inkontaktbringen der Proteine aus (a) und (b);
(d) Überwachen der Komplexbildung; und gegebenenfalls
(e) Bestimmen der Komplexstabilität oder der Kinetik mit bekannten *in vitro*-Verfahren.

7. Kit oder Testsystem, umfassend eine Zusammensetzung nach einem der Ansprüche 1 bis 3.

## Revendications

1. Composition comprenant la protéine virale NS3 issue de HCV ou des fragments peptidiques de celle-ci ayant une longueur d'au moins 6 résidus d'acide aminé et la protéine MARCH7 selon SEQ ID N° : 2 ou codée par SEQ ID N° : 4 ou des fragments peptidiques de celle-ci ayant une longueur d'au moins 6 résidus d'acide aminé, dans laquelle la protéine ou les fragments peptidiques de NS3 sont capables de lier ladite protéine MARCH7.

2. Composition selon la revendication 1, dans laquelle la protéine virale NS3 issue de HCV a une séquence selon SEQ ID N° : 5 ou SEQ ID N° : 15 ou est codée par une séquence selon SEQ ID N° : 6 ou SEQ ID N° : 16.

3. Composition selon la revendication 1 ou 2, dans laquelle les deux protéines ou peptides sont dans un complexe.

4. Procédé de criblage de composés, capables de moduler la formation d'un complexe et/ou la stabilité d'un complexe de la NS3 virale issue de HCV et de la MARCH7 selon SEQ ID N° : 2 ou codée par SEQ ID N° : 4 comprenant les étapes de :
(a) soumission d'un composé étudié à la composition selon l'une quelconque des revendications 1 à 3 ;
(b) suivi des changements de la formation d'un complexe des protéines ou des fragments peptidiques de la composition selon la revendication 1 ou 2 et/ou de la stabilité d'un complexe du complexe de la composition selon la revendication 3 ; et
(c) détermination d'un composé capable de moduler la formation et/ou la stabilité d'un complexe d'après sa capacité à modifier la formation d'un complexe entre les protéines de la composition selon la revendication 1 ou 2 et/ou de modifier la stabilité du complexe selon la revendication 3, par rapport à un témoin.

5. Procédé de criblage de composés, capables de moduler la formation d'un complexe et/ou la stabilité d'un complexe de la NS3 virale issue de HCV et de la MARCH7 selon SEQ ID N° : 2 ou codée par SEQ ID N° : 4 selon la revendication 4, dans lequel le composé étudié est identifié comme étant un composé étudié candidat avant l'étape (a) par
soumission d'un composé étudié à une composition comprenant une protéine MARCH7, ou la protéiné codée par SEQ ID N° : 9, ou des fragments de celle-ci ; et identification des composés qui sont capables de lier la MARCH7, ou la protéiné codée par SEQ ID N° : 9, ou des fragments de celle-ci.

6. Procédé de préparation, de détection et d'analyse du complexe entre la NS3 virale issue de HCV et la MARCH7 selon SEQ ID N° : 2 ou codée par SEQ ID N° : 4, comprenant les étapes de :
(a) synthèse et/ou isolement de la protéine virale NS3 de HCV ;
(b) synthèse et/ou isolement de la protéine MARCH7 humaine selon SEQ ID N° : 2 ou codée par SEQ ID N° : 4 ;
(c) mise en contact des protéines de (a) et de (b) ;
(d) suivi de la formation d'un complexe ; et éventuellement ;
(e) détermination de la stabilité d'un complexe ou de la cinétique par des procédés *in vitro* connus.

7. Kit ou système d'essai comprenant une composition selon l'une quelconque des revendications 1 à 3.


### SEQUENCE LISTING

<110> Nexigen GmbH
<120> Human HCV Interacting Proteins and Methods of Use
<130> R1992 PCT BLN
<160> 20
<170> PatentIn version 3.5
<210> 1
   <211> 591
   <212> PRT
   <213> Homo sapiens
<400> 1
   Met Ala Pro Glu Ile Asn Leu Pro Gly Pro Met Ser Leu Ile Asp Asn
   1               5                   10                  15
   Thr Lys Gly Gln Leu Val Val Asn Pro Glu Ala Leu Lys Ile Leu Ser
               20                  25                  30
   Ala Ile Thr Gln Pro Val Val Val Val Ala Ile Val Gly Leu Tyr Arg
           35                  40                  45
   Thr Gly Lys Ser Tyr Leu Met Asn Lys Leu Ala Gly Lys Lys Asn Gly
       50                  55                  60
   Phe Ser Leu Gly Ser Thr Val Lys Ser His Thr Lys Gly Ile Trp Met
   65                  70                  75                  80
   Trp Cys Val Pro His Pro Lys Lys Pro Glu His Thr Leu Val Leu Leu
                   85                  90                  95
   Asp Thr Glu Gly Leu Gly Asp Ile Glu Lys Gly Asp Asn Glu Asn Asp
               100                 105                 110
   Ser Trp Ile Phe Ala Leu Ala Ile Leu Leu Ser Ser Thr Phe Val Tyr
           115                 120                 125
   Asn Ser Met Gly Thr Ile Asn Gln Gln Ala Met Asp Gln Leu His Tyr
       130                 135                 140
   Val Thr Glu Leu Thr Asp Arg Ile Lys Ala Asn Ser Ser Pro Gly Asn
   145                 150                 155                 160
   Asn Ser Val Asp Asp Ser Ala Asp Phe Val Ser Phe Phe Pro Ala Phe
                   165                 170                 175
   Val Trp Thr Leu Arg Asp Phe Thr Leu Glu Leu Glu Val Asp Gly Glu
               180                 185                 190
   Pro Ile Thr Ala Asp Asp Tyr Leu Glu Leu Ser Leu Lys Leu Arg Lys
           195                 200                 205
   Gly Thr Asp Lys Lys Ser Lys Ser Phe Asn Asp Pro Arg Leu Cys Ile
       210                 215                 220
   Arg Lys Phe Phe Pro Lys Arg Lys Cys Phe Val Phe Asp Trp Pro Ala
   225                 230                 235                 240
   Pro Lys Lys Tyr Leu Ala His Leu Glu Gln Leu Lys Glu Glu Glu Leu
                   245                 250                 255
   Asn Pro Asp Phe Ile Glu Gln Val Ala Glu Phe Cys Ser Tyr Ile Leu
               260                 265                 270
   Ser His Ser Asn Val Lys Thr Leu Ser Gly Gly Ile Pro Val Asn Gly
           275                 280                 285
   Pro Arg Leu Glu Ser Leu Val Leu Thr Tyr Val Asn Ala Ile Ser Ser
       290                 295                 300
   Gly Asp Leu Pro Cys Met Glu Asn Ala Val Leu Ala Leu Ala Gln Ile
   305                 310                 315                 320
   Glu Asn Ser Ala Ala Val Glu Lys Ala Ile Ala His Tyr Glu Gln Gln
                   325                 330                 335
   Met Gly Gln Lys Val Gln Leu Pro Thr Glu Thr Leu Gln Glu Leu Leu
               340                 345                 350
   Asp Leu His Arg Asp Ser Glu Arg Glu Ala Ile Glu Val Phe Met Lys
           355                 360                 365
   Asn Ser Phe Lys Asp Val Asp Gln Met Phe Gln Arg Lys Leu Gly Ala
       370                 375                 380
   Gln Leu Glu Ala Arg Arg Asp Asp Phe Cys Lys Gln Asn Ser Lys Ala
   385                 390                 395                 400
   Ser Ser Asp Cys Cys Met Ala Leu Leu Gln Asp Ile Phe Gly Pro Leu
                   405                 410                 415
   Glu Glu Asp Val Lys Gln Gly Thr Phe Ser Lys Pro Gly Gly Tyr Arg
               420                 425                 430
   Leu Phe Thr Gln Lys Leu Gln Glu Leu Lys Asn Lys Tyr Tyr Gln Val
           435                 440                 445
   Pro Arg Lys Gly Ile Gln Ala Lys Glu Val Leu Lys Lys Tyr Leu Glu
       450                 455                 460
   Ser Lys Glu Asp Val Ala Asp Ala Leu Leu Gln Thr Asp Gln Ser Leu
   465 470 475 480
   Ser Glu Lys Glu Lys Ala Ile Glu Val Glu Arg Ile Lys Ala Glu Ser
   485 490 495
   Ala Glu Ala Ala Lys Lys Met Leu Glu Glu Ile Gln Lys Lys Asn Glu
   500 505 510
   Glu Met Met Glu Gln Lys Glu Lys Ser Tyr Gln Glu His Val Lys Gln
   515 520 525
   Leu Thr Glu Lys Met Glu Arg Asp Arg Ala Gln Leu Met Ala Glu Gln
   530 535 540
   Glu Lys Thr Leu Ala Leu Lys Leu Gln Glu Gln Glu Arg Leu Leu Lys
   545 550 555 560
   Glu Gly Phe Glu Asn Glu Ser Lys Arg Leu Gln Lys Asp Ile Trp Asp
   565 570 575
   Ile Gln Met Arg Ser Lys Ser Leu Glu Pro Ile Cys Asn Ile Leu
   580 585 590
<210> 2
   <211> 704
   <212> PRT
   <213> Homo sapiens
<400> 2
   Met Glu Ser Lys Pro Ser Arg Ile Pro Arg Arg Ile Ser Val Gln Pro
   1 5 10 15
   Ser Ser Ser Leu Ser Ala Arg Met Met Ser Gly Ser Arg Gly Ser Ser
   20 25 30
   Leu Asn Asp Thr Tyr His Ser Arg Asp Ser Ser Phe Arg Leu Asp Ser
   35 40 45
   Glu Tyr Gln Ser Thr Ser Ala Ser Ala Ser Ala Ser Pro Phe Gln Ser
   50 55 60
   Ala Trp Tyr Ser Glu Ser Glu Ile Thr Gln Gly Ala Arg Ser Arg Ser
   65 70 75 80
   Gln Asn Gln Gln Arg Asp His Asp Ser Lys Arg Pro Lys Leu Ser Cys
   85 90 95
   Thr Asn Cys Thr Thr Ser Ala Gly Arg Asn Val Gly Asn Gly Leu Asn
   100 105 110
   Thr Leu Ser Asp Ser Ser Trp Arg His Ser Gln Val Pro Arg Ser Ser
   115 120 125
   Ser Met Val Leu Gly Ser Phe Gly Thr Asp Leu Met Arg Glu Arg Arg
   130 135 140
   Asp Leu Glu Arg Arg Thr Asp Ser Ser Ile Ser Asn Leu Met Asp Tyr
   145 150 155 160
   Ser His Arg Ser Gly Asp Phe Thr Thr Ser Ser Tyr Val Gln Asp Arg
   165 170 175
   Val Pro Ser Tyr Ser Gln Gly Ala Arg Pro Lys Glu Asn Ser Met Ser
   180 185 190
   Thr Leu Gln Leu Asn Thr Ser Ser Thr Asn His Gln Leu Pro Ser Glu
   195 200 205
   His Gln Thr Ile Leu Ser Ser Arg Asp Ser Arg Asn Ser Leu Arg Ser
   210 215 220
   Asn Phe Ser Ser Arg Glu Ser Glu Ser Ser Arg Ser Asn Thr Gln Pro
   225 230 235 240
   Gly Phe Ser Tyr Ser Ser Ser Arg Asp Glu Ala Pro Ile Ile Ser Asn
   245 250 255
   Ser Glu Arg Val Val Ser Ser Gln Arg Pro Phe Gln Glu Ser Ser Asp
   260 265 270
   Asn Glu Gly Arg Arg Thr Thr Arg Arg Leu Leu Ser Arg Ile Ala Ser
   275 280 285
   Ser Met Ser Ser Thr Phe Phe Ser Arg Arg Ser Ser Gln Asp Ser Leu
   290 295 300
   Asn Thr Arg Ser Leu Asn Ser Glu Asn Ser Tyr Val Ser Pro Arg Ile
   305 310 315 320
   Leu Thr Ala Ser Gln Ser Arg Ser Asn Val Pro Ser Ala Ser Glu Val
   325 330 335
   Pro Asp Asn Arg Ala Ser Glu Ala Ser Gln Gly Phe Arg Phe Leu Arg
   340 345 350
   Arg Arg Trp Gly Leu Ser Ser Leu Ser His Asn His Ser Ser Glu Ser
   355 360 365
   Asp Ser Glu Asn Phe Asn Gln Glu Ser Glu Gly Arg Asn Thr Gly Pro
   370 375 380
   Trp Leu Ser Ser Ser Leu Arg Asn Arg Cys Thr Pro Leu Phe Ser Arg
   385 390 395 400
   Arg Arg Arg Glu Gly Arg Asp Glu Ser Ser Arg Ile Pro Thr Ser Asp
   405 410 415
   Thr Ser Ser Arg Ser His Ile Phe Arg Arg Glu Ser Asn Glu Val Val
   420 425 430
   His Leu Glu Ala Gln Asn Asp Pro Leu Gly Ala Ala Ala Asn Arg Pro
   435 440 445
   Gln Ala Ser Ala Ala Ser Ser Ser Ala Thr Thr Gly Gly Ser Thr Ser
   450 455 460
   Asp Ser Ala Gln Gly Gly Arg Asn Thr Gly Ile Ser Gly Ile Leu Pro
   465 470 475 480
   Gly Ser Leu Phe Arg Phe Ala Val Pro Pro Ala Leu Gly Ser Asn Leu
   485 490 495
   Thr Asp Asn Val Met Ile Thr Val Asp Ile Ile Pro Ser Gly Trp Asn
   500 505 510
   Ser Ala Asp Gly Lys Ser Asp Lys Thr Lys Ser Ala Pro Ser Arg Asp
   515 520 525
   Pro Glu Arg Leu Gln Lys Ile Lys Glu Ser Leu Leu Leu Glu Asp Ser
   530 535 540
   Glu Glu Glu Glu Gly Asp Leu Cys Arg Ile Cys Gln Met Ala Ala Ala
   545 550 555 560
   Ser Ser Ser Asn Leu Leu Ile Glu Pro Cys Lys Cys Thr Gly Ser Leu
   565 570 575
   Gln Tyr Val His Gln Asp Cys Met Lys Lys Trp Leu Gln Ala Lys Ile
   580 585 590
   Asn Ser Gly Ser Ser Leu Glu Ala Val Thr Thr Cys Glu Leu Cys Lys
   595 600 605
   Glu Lys Leu Glu Leu Asn Leu Glu Asp Phe Asp Ile His Glu Leu His
   610 615 620
   Arg Ala His Ala Asn Glu Gln Ala Glu Tyr Glu Phe Ile Ser Ser Gly
   625 630 635 640
   Leu Tyr Leu Val Val Leu Leu His Leu Cys Glu Gln Ser Phe Ser Asp
   645 650 655
   Met Met Gly Asn Thr Asn Glu Pro Ser Thr Arg Val Arg Phe Ile Asn
   660 665 670
   Leu Ala Arg Thr Leu Gln Ala His Met Glu Asp Leu Glu Thr Ser Glu
   675 680 685
   Asp Asp Ser Glu Glu Asp Gly Asp His Asn Arg Thr Phe Asp Ile Ala
   690 695 700
<210> 3
   <211> 1776
   <212> DNA
   <213> Homo sapiens
<400> 3
   atggctccag agatcaactt gccgggccca atgagcctca ttgataacac taaagggcag      60
   ctggtggtga atccagaagc tctgaagatc ctatctgcaa ttacgcagcc tgtggtggtg     120
   gtggcgattg tgggcctcta tcgcacaggc aaatcctacc tgatgaacaa gctggctggg     180
   aagaaaaacg gcttctctct aggctccaca gtgaagtctc acaccaaggg aatctggatg     240
   tggtgtgtgc ctcatcccaa gaagccagaa cacaccctag ttctgctcga cactgagggc     300
   ctgggagata tagagaaggg tgacaatgag aatgactcct ggatctttgc cttggccatc     360
   ctcctgagca gcaccttcgt gtacaatagc atgggaacca tcaaccagca ggccatggac     420
   caacttcact atgtgacaga gctgacagat cgaatcaagg caaactcctc acctggtaac     480
   aattctgtag acgactcagc tgactttgtg agcttttttc cagcatttgt gtggactctc     540
   agagatttca ccctggaact ggaagtagat ggagaaccca tcactgctga tgactacttg     600
   gagctttcgc taaagctaag aaaaggtact gataagaaaa gtaaaagctt taatgatcct     660
   cggttgtgca tccgaaagtt cttccccaag aggaagtgct tcgtcttcga ttggcccgct     720
   cctaagaagt accttgctca cctagagcag ctaaaggagg aagagctgaa ccctgatttc     780
   atagaacaag ttgcagaatt ttgttcctac atcctcagcc attccaatgt caagactctt     840
   tcaggtggca ttccagtcaa tgggcctcgt ctagagagcc tggtgctgac ctacgtcaat     900
   gccatcagca gtggggatct accctgcatg gagaacgcag tcctggcctt ggcccagata     960
   gagaactcag ccgcagtgga aaaggctatt gcccactatg aacagcagat gggccagaag    1020
   gtgcagctgc ccacggaaac cctccaggag ctgctggacc tgcacaggga cagtgagaga    1080
   gaggccattg aagtcttcat gaagaactct ttcaaggatg tggaccaaat gttccagagg    1140
   aaattagggg cccagttgga agcaaggcga gatgactttt gtaagcagaa ttccaaagca    1200
   tcatcagatt gttgcatggc tttacttcag gatatatttg gccctttaga agaagatgtc    1260
   aagcagggaa cattttctaa accaggaggt taccgtctct ttactcagaa gctgcaggag    1320
   ctgaagaata agtactacca ggtgccaagg aaggggatac aggccaaaga ggtgctgaaa    1380
   aaatatttgg agtccaagga ggatgtggct gatgcacttc tacagactga tcagtcactc    1440
   tcagaaaagg aaaaagcgat tgaagtggaa cgtataaagg ctgaatctgc agaagctgca    1500
   aagaaaatgt tggaggaaat acaaaagaag aatgaggaga tgatggaaca gaaagagaag    1560
   agttatcagg aacatgtgaa acaattgact gagaagatgg agagggacag ggcccagtta 1620
   atggcagagc aagagaagac cctcgctctt aaacttcagg aacaggaacg ccttctcaag 1680
   gagggattcg agaatgagag caagagactt caaaaagaca tatgggatat ccagatgaga 1740
   agcaaatcat tggagccaat atgtaacata ctctaa 1776
<210> 4
   <211> 2115
   <212> DNA
   <213> Homo sapiens
<400> 4
   atggagtcta aaccttcaag gattccaaga agaatttctg ttcaaccttc cagctcctta 60
   agtgctagga tgatgtctgg aagcagagga agtagtttaa atgataccta tcactcaaga 120
   gactcttcat ttagattgga ttctgaatat cagtctacat cagcatcagc atctgcgtca 180
   ccatttcaat ctgcatggta tagtgaatct gagataactc agggagcacg ctcaagatcg 240
   cagaaccagc aacgggatca tgattcaaaa agacctaaac tttcctgtac aaactgtact 300
   acctcagctg ggagaaatgt tggaaatggt ttaaacacat tatcagattc atcttggagg 360
   catagtcaag ttcctagatc ttcatcaatg gtacttggat catttggaac agacttaatg 420
   agagagagga gagatttgga gagaagaaca gattcctcta ttagtaatct tatggattat 480
   agtcaccgaa gtggtgattt cacaacttca tcatatgttc aagacagagt tccttcatat 540
   tcacaaggag caagaccaaa agaaaactca atgagcactt tacagttgaa tacatcatcc 600
   acaaaccacc aattgccttc tgaacatcag accatactaa gttctaggga ctccagaaat 660
   tctttaagat caaatttttc ttcaagagaa tcagaatctt cccgaagcaa tacgcagcct 720
   ggattttctt acagttcaag tagagatgaa gccccaatca taagcaattc agaaagggtt 780
   gtttcatctc aaagaccatt tcaagaatct tctgacaatg aaggtaggcg gacaacgagg 840
   agattgctgt cacgcatagc ttctagcatg tcatctactt ttttttcacg aagatctagt 900
   caggattcct tgaatacaag atcattgaat tctgaaaatt cttacgtttc tccaagaatc 960
   ttgacagctt cacagtcccg tagtaatgta ccatcagctt ctgaagttcc cgataatagg 1020
   gcatctgaag cttctcaggg atttcgattt cttaggcgaa gatggggttt gtcatctctt 1080
   agccacaatc atagctctga gtcagattca gaaaatttta accaagaatc tgaaggtaga 1140
   aatacaggac catggttatc ttcctcactt agaaatagat gcacaccttt gttctctaga 1200
   aggaggcgag agggaagaga tgaatcttca aggataccta cctctgatac atcatctaga 1260
   tctcatattt ttagaagaga atcaaatgaa gtggttcacc ttgaagcaca gaatgatcct 1320
   cttggagctg ctgccaacag accacaagca tctgcagcat caagcagtgc cacaacaggt 1380
   ggctctacat cagattcggc tcaaggtgga agaaatacag gaatatcagg gattcttcct 1440
   ggttccttat tccggtttgc agtcccccca gcacttggga gtaatttgac cgacaatgtc 1500
   atgatcacag tagatattat tccttcaggt tggaattcag ctgatggtaa aagtgataaa 1560
   actaaaagtg cgccttcaag agatccagaa agattgcaga aaataaaaga gagcctcctt 1620
   ttagaggact cagaagaaga agaaggtgac ttatgtagaa tttgtcaaat ggcagctgca 1680
   tcatcatcta atttgctgat agagccatgc aagtgcacag gaagtttgca gtatgtccac 1740
   caagactgta tgaaaaagtg gttacaggcc aaaattaact ctggttcttc attagaagct 1800
   gtaaccacct gtgaactatg taaagagaag ttggagctta acctggagga ttttgatatt 1860
   catgaactac atagagctca tgcaaatgaa caagctgagt atgagtttat cagctctggt 1920
   ctctacctag tggtgttatt gcacttgtgc gaacaaagct tttctgatat gatgggaaat 1980
   acaaatgaac caagcacacg tgtccggttt attaaccttg caagaactct tcaggcacat 2040
   atggaagatc tcgaaacttc agaggatgat tccgaagaag acggagacca taacaggaca 2100
   tttgatattg cctaa 2115
<210> 5
   <211> 632
   <212> PRT
   <213> Hepatitis C virus
<400> 5
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Thr Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ser Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Ser Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Leu Phe Arg Ala Ala Val Cys
   145 150 155 160
   Thr Arg Gly Val Thr Lys Ala Val Asp Phe Ile Pro Val Glu Asn Leu
                   165                 170                 175
   Glu Thr Thr Met Arg Ser Pro Val Phe Thr Asp Asn Ser Ser Pro Pro
               180                 185                 190
   Ala Val Pro Gln Ser Phe Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Leu Gly Phe Gly
   225                 230                 235                 240
   Ala Tyr Met Ser Lys Ala His Gly Val Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Val Val
   305                 310                 315                 320
   Leu Ala Thr Ala Thr Pro Pro Gly Ser Val Thr Val Ser His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Val Ile Lys Arg Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Ile Asn Ala Val Ala Tyr Tyr Arg Gly Leu Asp Val Ser Val
   385                 390                 395                 400
   Ile Pro Thr Asn Gly Asp Val Val Val Val Ser Thr Asp Ala Leu Met
                   405                 410                 415
   Thr Gly Phe Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
   435 440 445
   Thr Thr Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Met Pro Ala Glu Thr Thr Val
   500 505 510
   Arg Leu Arg Ala Tyr Met Asn Thr Pro Gly Leu Pro Val Cys Gln Asp
   515 520 525
   His Leu Glu Phe Trp Glu Gly Val Phe Thr Gly Leu Thr His Ile Asp
   530 535 540
   Ala His Phe Leu Ser Gln Thr Lys Gln Ser Gly Glu Asn Phe Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Val Thr Leu Thr His Pro Ile Thr Lys Tyr Ile Met Thr Cys Met
   610 615 620
   Ser Ala Asp Leu Glu Val Val Thr
   625 630
<210> 6
   <211> 1893
   <212> DNA
   <213> Hepatitis C virus
<400> 6
   gcgcccatca cggcgtacgc ccagcagacg agaggcctcc tagggtgtat aatcaccagc 60
   ctgactggcc gggacaaaaa ccaagtggag ggtgaggtcc agatcgtgtc aactgctacc 120
   caaaccttcc tggcaacgtg catcaatggg gtatgctgga ctgtctacca cggggccgga 180
   acgaggacca tcgcatcacc caagggtcct gtcatccaga tgtataccaa tgtggaccaa 240
   gaccttgtgg gctggcccgc tcctcaaggt tcccgctcat tgacaccctg cacctgcggc 300
   tcctcggacc tttacctggt tacgaggcac gccgacgtca ttcccgtgcg ccggcgaggt     360
   gatagcaggg gtagcctgct ttcgccccgg cccatttcct acctaaaagg ctcctcgggg     420
   ggtccgctgt tgtgccccgc gggacacgcc gtgggcctat tcagggccgc ggtgtgcacc     480
   cgtggagtga ccaaggcggt ggactttatc cctgtggaga acctagagac aaccatgaga     540
   tccccggtgt tcacggacaa ctcctctcca ccagcagtgc cccagagctt ccaggtggcc     600
   cacctgcatg ctcccaccgg cagtggtaag agcaccaagg tcccggctgc gtacgcagcc     660
   cagggctaca aggtgttggt gctcaacccc tctgttgctg caacgctggg ctttggtgct     720
   tacatgtcca aggcccatgg ggtcgatcct aatatcagga ccggggtgag aacaattacc     780
   actggcagcc ccatcacgta ctccacctac ggcaagttcc ttgccgacgg cgggtgctca     840
   ggaggcgctt atgacataat aatttgtgac gagtgccact ccacggatgc cacatccatc     900
   ttgggcatcg gcactgtcct tgaccaagca gagactgcgg gggcgagatt ggttgtgctc     960
   gccactgcta cccctccggg ctccgtcact gtgtcccatc ctaacatcga ggaggttgct    1020
   ctgtccacca ccggagagat ccctttctac ggcaaggcta tccccctcga ggtgatcaag    1080
   aggggaagac atctcatctt ctgtcactca aagaagaagt gcgacgagct cgccgcgaag    1140
   ctggtcgcat tgggcatcaa tgccgtggcc tactaccgcg gacttgacgt gtctgtcatc    1200
   ccgaccaacg gcgatgttgt cgtcgtgtcg accgatgctc tcatgactgg ctttaccggc    1260
   gacttcgact ctgtgataga ctgcaacacg tgtgtcactc agacagtcga tttcagcctt    1320
   gaccctacct ttaccattga gacaaccacg ctcccccagg atgctgtctc caggactcag    1380
   cgccggggca ggactggcag ggggaagcca ggcatctaca gatttgtggc accgggggag    1440
   cgcccctccg gcatgttcga ctcgtccgtc ctctgtgagt gctatgacgc gggctgtgct    1500
   tggtatgagc tcatgcccgc cgagactaca gttaggctac gagcgtacat gaacaccccg    1560
   gggcttcccg tgtgccagga ccatcttgaa ttttgggagg gcgtctttac gggcctcacc    1620
   catatagatg cccactttct atcccagaca aagcagagtg gggagaactt tccttacctg    1680
   gtagcgtacc aagccaccgt gtgcgctagg gctcaagccc ctcccccatc gtgggaccag    1740
   atgtggaagt gtttgatccg ccttaaaccc accctccatg ggccaacacc cctgctatac    1800
   agactgggcg ctgttcagaa tgaagtcacc ctgacgcacc caatcaccaa atacatcatg    1860
   acatgcatgt cggccgacct ggaggtcgtc aca                                 1893
<210> 7
   <211> 10
   <212> PRT
   <213> Artificial
<220>
   <223> linker
<400> 7
   Gly Gly Gly Gly Ser Gly Gly Gly Gly Ser
   1               5                   10
<210> 8
   <211> 465
   <212> PRT
   <213> Hepatitis C virus
<400> 8
   Val Asp Phe Ile Pro Val Glu Asn Leu Glu Thr Thr Met Arg Ser Pro
   1               5                   10                  15
   Val Phe Thr Asp Asn Ser Ser Pro Pro Ala Val Pro Gln Ser Phe Gln
               20                  25                  30
   Val Ala His Leu His Ala Pro Thr Gly Ser Gly Lys Ser Thr Lys Val
           35                  40                  45
   Pro Ala Ala Tyr Ala Ala Gln Gly Tyr Lys Val Leu Val Leu Asn Pro
       50                  55                  60
   Ser Val Ala Ala Thr Leu Gly Phe Gly Ala Tyr Met Ser Lys Ala His
   65                  70                  75                  80
   Gly Val Asp Pro Asn Ile Arg Thr Gly Val Arg Thr Ile Thr Thr Gly
                   85                  90                  95
   Ser Pro Ile Thr Tyr Ser Thr Tyr Gly Lys Phe Leu Ala Asp Gly Gly
               100                 105                 110
   Cys Ser Gly Gly Ala Tyr Asp Ile Ile Ile Cys Asp Glu Cys His Ser
           115                 120                 125
   Thr Asp Ala Thr Ser Ile Leu Gly Ile Gly Thr Val Leu Asp Gln Ala
       130                 135                 140
   Glu Thr Ala Gly Ala Arg Leu Val Val Leu Ala Thr Ala Thr Pro Pro
   145                 150                 155                 160
   Gly Ser Val Thr Val Ser His Pro Asn Ile Glu Glu Val Ala Leu Ser
                   165                 170                 175
   Thr Thr Gly Glu Ile Pro Phe Tyr Gly Lys Ala Ile Pro Leu Glu Val
               180                 185                 190
   Ile Lys Gly Gly Arg His Leu Ile Phe Cys His Ser Lys Lys Lys Cys
           195                 200                 205
   Asp Glu Leu Ala Ala Lys Leu Val Ala Leu Gly Ile Asn Ala Val Ala
       210                 215                 220
   Tyr Tyr Arg Gly Leu Asp Val Ser Val Ile Pro Thr Asn Gly Asp Val
   225                 230                 235                 240
   Val Val Val Ser Thr Asp Ala Leu Met Thr Gly Phe Thr Gly Asp Phe
   245 250 255
   Asp Ser Val Ile Asp Cys Asn Thr Cys Val Thr Gln Thr Val Asp Phe
   260 265 270
   Ser Leu Asp Pro Thr Phe Thr Ile Glu Thr Thr Thr Leu Pro Gln Asp
   275 280 285
   Ala Val Ser Arg Thr Gln Arg Arg Gly Arg Thr Gly Arg Gly Lys Pro
   290 295 300
   Gly Ile Tyr Arg Phe Val Ala Pro Gly Glu Arg Pro Ser Gly Met Phe
   305 310 315 320
   Asp Ser Ser Val Leu Cys Glu Cys Tyr Asp Ala Gly Cys Ala Trp Tyr
   325 330 335
   Glu Leu Met Pro Ala Glu Thr Thr Val Arg Leu Arg Ala Tyr Met Asn
   340 345 350
   Thr Pro Gly Leu Pro Val Cys Gln Asp His Leu Glu Phe Trp Glu Gly
   355 360 365
   Val Phe Thr Gly Leu Thr His Ile Asp Ala His Phe Leu Ser Gln Thr
   370 375 380
   Lys Gln Ser Gly Glu Asn Phe Pro Tyr Leu Val Ala Tyr Gln Ala Thr
   385 390 395 400
   Val Cys Ala Arg Ala Gln Ala Pro Pro Pro Ser Trp Asp Gln Met Trp
   405 410 415
   Lys Cys Leu Ile Arg Leu Lys Pro Thr Leu His Gly Pro Thr Pro Leu
   420 425 430
   Leu Tyr Arg Leu Gly Ala Val Gln Asn Glu Val Thr Leu Thr His Pro
   435 440 445
   Ile Thr Lys Tyr Ile Met Thr Cys Met Ser Ala Asp Leu Glu Val Val
   450 455 460
   Thr
   465
<210> 9
   <211> 1395
   <212> DNA
   <213> Hepatitis C virus
<400> 9
   gtggacttta tccctgtgga gaacctagag acaaccatga gatccccggt gttcacggac 60
   aactcctctc caccagcagt gccccagagc ttccaggtgg cccacctgca tgctcccacc 120
   ggcagtggta agagcaccaa ggtcccggct gcgtacgcag cccagggcta caaggtgttg 180
   gtgctcaacc cctctgttgc tgcaacgctg ggctttggtg cttacatgtc caaggcccat 240
   ggggtcgatc ctaatatcag gaccggggtg agaacaatta ccactggcag ccccatcacg 300
   tactccacct acggcaagtt ccttgccgac ggcgggtgct caggaggcgc ttatgacata 360
   ataatttgtg acgagtgcca ctccacggat gccacatcca tcttgggcat cggcactgtc 420
   cttgaccaag cagagactgc gggggcgaga ttggttgtgc tcgccactgc tacccctccg 480
   ggctccgtca ctgtgtccca tcctaacatc gaggaggttg ctctgtccac caccggagag 540
   atccctttct acggcaaggc tatccccctc gaggtgatca aggggggaag acatctcatc 600
   ttctgtcact caaagaagaa gtgcgacgag ctcgccgcga agctggtcgc attgggcatc 660
   aatgccgtgg cctactaccg cggacttgac gtgtctgtca tcccgaccaa cggcgatgtt 720
   gtcgtcgtgt cgaccgatgc tctcatgact ggctttaccg gcgacttcga ctctgtgata 780
   gactgcaaca cgtgtgtcac tcagacagtc gatttcagcc ttgaccctac ctttaccatt 840
   gagacaacca cgctccccca ggatgctgtc tccaggactc agcgccgggg caggactggc 900
   agggggaagc caggcatcta cagatttgtg gcaccggggg agcgcccctc cggcatgttc 960
   gactcgtccg tcctctgtga gtgctatgac gcgggctgtg cttggtatga gctcatgccc 1020
   gccgagacta cagttaggct acgagcgtac atgaacaccc cggggcttcc cgtgtgccag 1080
   gaccatcttg aattttggga gggcgtcttt acgggcctca cccatataga tgcccacttt 1140
   ctatcccaga caaagcagag tggggagaac tttccttacc tggtagcgta ccaagccacc 1200
   gtgtgcgcta gggctcaagc ccctccccca tcgtgggacc agatgtggaa gtgtttgatc 1260
   cgccttaaac ccaccctcca tgggccaaca cccctgctat acagactggg cgctgttcag 1320
   aatgaagtca ccctgacgca cccaatcacc aaatacatca tgacatgcat gtcggccgac 1380
   ctggaggtcg tcaca 1395
<210> 10
   <211> 41
   <212> DNA
   <213> Artificial
<220>
   <223> primer
<400> 10
   atcaactcca agcttaccat ggcgcccatc acggcgtacg c 41
<210> 11
   <211> 39
   <212> DNA
   <213> Artificial
<220>
   <223> primer
<400> 11
   gcccccggga agctttgtga cgacctccag gtcggccga 39
<210> 12
   <211> 32
   <212> DNA
   <213> Artificial
<220>
   <223> primer
<400> 12
   aaatatgcgg ccgcctatgt gacgacctcc ag 32
<210> 13
   <211> 27
   <212> DNA
   <213> Artificial
<220>
   <223> primer
<400> 13
   ttttcggacc ggtggacttt atccctg 27
<210> 14
   <211> 3484
   <212> DNA
   <213> Homo sapiens
<400> 14
   ggtggctggt tctgcgccgg atccgggaga ggggcgggcg ccattgtgct tcgctgccga      60
   ctgcatttcc tcagtcacgg gcctagaact ccaaggagaa aggcggcgaa aaatctttaa     120
   gaatggagtc taaaccttca aggattccaa gaagaatttc tgttcaacct tccagctcct     180
   taagtgctag gatgatgtct ggaagcagag gaagtagttt aaatgatacc tatcactcaa     240
   gagactcttc atttagattg gattctgaat atcagtctac atcagcatca gcatctgcgt     300
   caccatttca atctgcatgg tatagtgaat ctgagataac tcagggagca cgctcaagat     360
   cgcagaacca gcaacgggat catgattcaa aaagacctaa actttcctgt acaaactgta     420
   ctacctcagc tgggagaaat gttggaaatg gtttaaacac attatcagat tcatcttgga     480
   ggcatagtca agttcctaga tcttcatcaa tggtacttgg atcatttgga acagacttaa     540
   tgagagagag gagagatttg gagagaagaa cagattcctc tattagtaat cttatggatt     600
   atagtcaccg aagtggtgat ttcacaactt catcatatgt tcaagacaga gttccttcat     660
   attcacaagg agcaagacca aaagaaaact caatgagcac tttacagttg aatacatcat     720
   ccacaaacca ccaattgcct tctgaacatc agaccatact aagttctagg gactccagaa     780
   attctttaag atcaaatttt tcttcaagag aatcagaatc ttcccgaagc aatacgcagc     840
   ctggattttc ttacagttca agtagagatg aagccccaat cataagcaat tcagaaaggg     900
   ttgtttcatc tcaaagacca tttcaagaat cttctgacaa tgaaggtagg cggacaacga     960
   ggagattgct gtcacgcata gcttctagca tgtcatctac ttttttttca cgaagatcta    1020
   gtcaggattc cttgaataca agatcattga attctgaaaa ttcttacgtt tctccaagaa    1080
   tcttgacagc ttcacagtcc cgtagtaatg taccatcagc ttctgaagtt cccgataata 1140
   gggcatctga agcttctcag ggatttcgat ttcttaggcg aagatggggt ttgtcatctc 1200
   ttagccacaa tcatagctct gagtcagatt cagaaaattt taaccaagaa tctgaaggta 1260
   gaaatacagg accatggtta tcttcctcac ttagaaatag atgcacacct ttgttctcta 1320
   gaaggaggcg agagggaaga gatgaatctt caaggatacc tacctctgat acatcatcta 1380
   gatctcatat ttttagaaga gaatcaaatg aagtggttca ccttgaagca cagaatgatc 1440
   ctcttggagc tgctgccaac agaccacaag catctgcagc atcaagcagt gccacaacag 1500
   gtggctctac atcagattcg gctcaaggtg gaagaaatac aggaatatca gggattcttc 1560
   ctggttcctt attccggttt gcagtccctc cagcacttgg gagtaatttg accgacaatg 1620
   tcatgatcac agtagatatt attccttcag gttggaattc agctgatggt aaaagtgata 1680
   aaactaaaag tgcgccttca agagatccag aaagattgca gaaaataaaa gagagcctcc 1740
   ttttagagga ctcagaagaa gaagaaggtg acttatgtag aatttgtcaa atggcagctg 1800
   catcatcatc taatttgctg atagagccat gcaagtgcac aggaagtttg cagtatgtcc 1860
   accaagactg tatgaaaaag tggttacagg ccaaaattaa ctctggttct tcattagaag 1920
   ctgtaaccac ctgtgaacta tgtaaagaga agttggagct taacctggag gattttgata 1980
   ttcatgaact acatagagct catgcaaatg aacaagctga gtatgagttt atcagctctg 2040
   gtctctacct agtggtgtta ttgcacttgt gcgaacaaag cttttctgat atgatgggaa 2100
   atacaaatga accaagcaca cgtgtccgat ttattaacct tgcaagaact cttcaggcac 2160
   atatggaaga tctcgaaact tcagaggatg attccgaaga agacggagac cataacagga 2220
   catttgatat tgcctaactt catataagac agatggatga tctgtgaaca taagtgttta 2280
   ttaaaaatgg caattaaata taaattactt ttgtggggga atgcctaata aatacattga 2340
   ctatatataa aatgaatata tacatacaca tgtatgcctg tatatatata ttcattctcc 2400
   agtgttgctg aattaaaatt ctgctggact ttttaacata gcaaatccga tgtttataaa 2460
   ctggtaatca aaaaggtttt ttcttttagg tgagtgggaa agtattaccc ttgttttaaa 2520
   tatctaagca atgcctatca accctttttt gtgttatgat tactgtagtc atatttatga 2580
   aaaaaggttt gtgttttact cttgctagtg agaaaagtgg gacaaaatat acttttgaaa 2640
   taaaatgcta tatggcacct aattattttt tcttttaaaa tgccttaagt tgcagtctca 2700
   ttttgataat catttgcttc cagtgtttaa aaattaaaaa aagaatgggg agaaggttat 2760
   gagaagagca ttattaagtt tccaaattta atttgaattc caaattcacc tagcaataaa 2820
   atctaatttt taaaaagtat ataaatataa aatgtataaa tgatggatag atttttgtat 2880
   tgatttgcaa aatgcagatt atatttgata ggctatagta tgtagatatt ccttttagga 2940
   atattacagc tgtaaattat atgagacttg ccagtcaaat gctatttggt ttaaaaaaat 3000
   tattgcaatc tcaagttaat ggaatatttt taaatcccac attcagagtt taaaacactg 3060
   gttttcaatg tgttttttag tgttgtcact tgtttataga taaatatata aataacctgt 3120
   ttggatcctg gtccttttta actgttcctt ggtaattctg agcatttatt tgatgactta    3180
   atatttttca ctacctttgg agaacagatg aacattattc accatgaatg gatctatact     3240
   gtgtggtcat gagttgtgta tacttccata acactgtatt tttcttctgt cagtaccctt    3300
   aggatacact ttaaaacacc ttaaggtctg atgttatggc aacaaactac tttttcaaac     3360
   ctaaatagga accatgtaat ttctcaaaag tgattgaaca gtttgcccac acttagtttg     3420
   ttggtcttat gtaaaacatt ggctcaaaat aaagtacaca ctgatttaaa aaaaaaaaaa     3480
   aaaa                                                                  3484
<210> 15
   <211> 631
   <212> PRT
   <213> Hepatitis c virus
<400> 15
   Ala Pro Ile Thr Ala Tyr Ala Gln Gln Thr Arg Gly Leu Leu Gly Cys
   1               5                   10                  15
   Ile Ile Thr Ser Leu Thr Gly Arg Asp Lys Asn Gln Val Glu Gly Glu
               20                  25                  30
   Val Gln Ile Val Ser Thr Ala Thr Gln Thr Phe Leu Ala Thr Cys Ile
           35                  40                  45
   Asn Gly Val Cys Trp Thr Val Tyr His Gly Ala Gly Thr Arg Thr Ile
       50                  55                  60
   Ala Ser Pro Lys Gly Pro Val Ile Gln Met Tyr Thr Asn Val Asp Gln
   65                  70                  75                  80
   Asp Leu Val Gly Trp Pro Ala Pro Gln Gly Ser Arg Ser Leu Thr Pro
                   85                  90                  95
   Cys Thr Cys Gly Ser Ser Asp Leu Tyr Leu Val Thr Arg His Ala Asp
               100                 105                 110
   Val Ile Pro Val Arg Arg Arg Gly Asp Ser Arg Gly Ser Leu Leu Ser
           115                 120                 125
   Pro Arg Pro Ile Ser Tyr Leu Lys Gly Ser Ser Gly Gly Pro Leu Leu
       130                 135                 140
   Cys Pro Ala Gly His Ala Val Gly Leu Phe Arg Ala Ala Val Cys Thr
   145                 150                 155                 160
   Arg Gly Val Ala Lys Ala Val Asp Phe Ile Pro Val Glu Asn Leu Gly
                   165                 170                 175
   Thr Thr Met Arg Ser Pro Val Phe Thr Asp Asn Ser Ser Pro Pro Ala
   180 185 190
   Val Pro Gln Ser Phe Gln Val Ala His Leu His Ala Pro Thr Gly Ser
   195 200 205
   Gly Lys Ser Thr Lys Val Pro Ala Ala Tyr Ala Ala Gln Gly Tyr Lys
   210 215 220
   Val Leu Val Leu Asn Pro Ser Val Ala Ala Thr Leu Gly Phe Gly Ala
   225 230 235 240
   Tyr Met Ser Lys Ala His Gly Val Asp Pro Asn Ile Arg Thr Gly Val
   245 250 255
   Arg Thr Ile Thr Thr Gly Ser Pro Ile Thr Tyr Ser Thr Tyr Gly Lys
   260 265 270
   Phe Leu Ala Asp Gly Gly Cys Ser Gly Gly Ala Tyr Asp Ile Ile Ile
   275 280 285
   Cys Asp Glu Cys His Ser Thr Asp Ala Thr Ser Ile Leu Gly Ile Gly
   290 295 300
   Thr Val Leu Asp Gln Ala Glu Thr Ala Gly Ala Arg Leu Val Val Leu
   305 310 315 320
   Ala Thr Ala Thr Pro Pro Gly Ser Val Thr Val Ser His Pro Asn Ile
   325 330 335
   Glu Glu Val Ala Leu Ser Thr Thr Gly Glu Ile Pro Phe Tyr Gly Lys
   340 345 350
   Ala Ile Pro Leu Glu Val Ile Lys Gly Gly Arg His Leu Ile Phe Cys
   355 360 365
   His Ser Lys Lys Lys Cys Asp Glu Leu Ala Ala Lys Leu Val Ala Leu
   370 375 380
   Gly Ile Asn Ala Val Ala Tyr Tyr Arg Gly Leu Asp Val Ser Val Ile
   385 390 395 400
   Pro Thr Ser Gly Asp Val Val Val Val Ser Thr Asp Ala Leu Met Thr
   405 410 415
   Gly Phe Thr Gly Asp Phe Asp Ser Val Ile Asp Cys Asn Thr Cys Val
   420 425 430
   Thr Gln Thr Val Asp Phe Ser Leu Asp Pro Thr Phe Thr Ile Glu Thr
   435 440 445
   Thr Thr Leu Pro Gln Asp Ala Val Ser Arg Thr Gln Arg Arg Gly Arg
   450 455 460
   Thr Gly Arg Gly Lys Pro Gly Ile Tyr Arg Phe Val Ala Pro Gly Glu
   465 470 475 480
   Arg Pro Ser Gly Met Phe Asp Ser Ser Val Leu Cys Glu Cys Tyr Asp
   485 490 495
   Ala Gly Cys Ala Trp Tyr Glu Leu Thr Pro Ala Glu Thr Thr Val Arg
   500 505 510
   Leu Arg Ala Tyr Met Asn Thr Pro Gly Leu Pro Val Cys Gln Asp His
   515 520 525
   Leu Glu Phe Trp Glu Gly Val Phe Thr Gly Leu Thr His Ile Asp Ala
   530 535 540
   His Phe Leu Ser Gln Thr Lys Gln Ser Gly Glu Asn Phe Pro Tyr Leu
   545 550 555 560
   Val Ala Tyr Gln Ala Thr Val Cys Ala Arg Ala Gln Ala Pro Pro Pro
   565 570 575
   Ser Trp Asp Gln Met Trp Lys Cys Leu Ile Arg Leu Lys Pro Thr Leu
   580 585 590
   His Gly Pro Thr Pro Leu Leu Tyr Arg Leu Gly Ala Val Gln Asn Glu
   595 600 605
   Val Thr Leu Thr His Pro Ile Thr Lys Tyr Ile Met Thr Cys Met Ser
   610 615 620
   Ala Asp Leu Glu Val Val Thr
   625 630
<210> 16
   <211> 1893
   <212> DNA
   <213> Hepatitis c virus
<400> 16
<210> 17
   <211> 465
   <212> PRT
   <213> Hepatitis C virus
<400> 17
   Val Asp Phe Ile Pro Val Gly Asn Leu Gly Thr Thr Met Arg Ser Pro
   1               5                   10                  15
   Val Phe Thr Asp Asn Ser Ser Pro Pro Ala Val Pro Gln Ser Phe Gln
               20                  25                  30
   Val Ala His Leu His Ala Pro Thr Gly Ser Gly Lys Ser Thr Lys Val
           35                  40                  45
   Pro Ala Ala Tyr Ala Ala Gln Gly Tyr Lys Val Leu Val Leu Asn Pro
   50 55 60
   Ser Val Ala Ala Thr Leu Gly Phe Gly Ala Tyr Met Ser Lys Ala His
   65 70 75 80
   Gly Val Asp Pro Asn Ile Arg Thr Gly Val Arg Thr Ile Thr Thr Gly
   85 90 95
   Ser Pro Ile Thr Tyr Ser Thr Tyr Gly Lys Phe Leu Ala Asp Gly Gly
   100 105 110
   Cys Ser Gly Gly Ala Tyr Asp Ile Ile Ile Cys Asp Glu Cys His Ser
   115 120 125
   Thr Asp Ala Thr Ser Ile Leu Gly Ile Gly Thr Val Leu Asp Gln Ala
   130 135 140
   Glu Thr Ala Gly Ala Arg Leu Val Val Leu Ala Thr Ala Thr Pro Pro
   145 150 155 160
   Gly Ser Val Thr Val Ser His Pro Asn Ile Glu Glu Val Ala Leu Ser
   165 170 175
   Thr Thr Gly Glu Ile Pro Phe Tyr Gly Lys Ala Ile Pro Leu Glu Val
   180 185 190
   Ile Lys Gly Gly Arg His Leu Ile Phe Cys His Ser Lys Lys Lys Cys
   195 200 205
   Asp Glu Leu Ala Ala Lys Leu Val Ala Leu Gly Ile Asn Ala Val Ala
   210 215 220
   Tyr Tyr Arg Gly Leu Asp Val Ser Val Ile Pro Thr Ser Gly Asp Val
   225 230 235 240
   Val Val Val Ser Thr Asp Ala Leu Met Thr Gly Phe Thr Gly Asp Phe
   245 250 255
   Asp Ser Val Ile Asp Cys Asn Thr Cys Val Thr Gln Thr Val Asp Phe
   260 265 270
   Ser Leu Asp Pro Thr Phe Thr Ile Glu Thr Thr Thr Leu Pro Gln Asp
   275 280 285
   Ala Val Ser Arg Thr Gln Arg Arg Gly Arg Thr Gly Arg Gly Lys Pro
   290 295 300
   Gly Ile Tyr Arg Phe Val Ala Pro Gly Glu Arg Pro Ser Gly Met Phe
   305 310 315 320
   Asp Ser Ser Val Leu Cys Glu Cys Tyr Asp Ala Gly Cys Ala Trp Tyr
   325 330 335
   Glu Leu Thr Pro Ala Glu Thr Thr Val Arg Leu Arg Ala Tyr Met Asn
   340 345 350
   Thr Pro Gly Leu Pro Val Cys Gln Asp His Leu Glu Phe Trp Glu Gly
   355 360 365
   Val Phe Thr Gly Leu Thr His Ile Asp Ala His Phe Leu Ser Gln Thr
   370 375 380
   Lys Gln Ser Gly Glu Asn Phe Pro Tyr Leu Val Ala Tyr Gln Ala Thr
   385 390 395 400
   Val Cys Ala Arg Ala Gln Ala Pro Pro Pro Ser Trp Asp Gln Met Trp
   405 410 415
   Lys Cys Leu Ile Arg Leu Lys Pro Thr Leu His Gly Pro Thr Pro Leu
   420 425 430
   Leu Tyr Arg Leu Gly Ala Val Gln Asn Glu Val Thr Leu Thr His Pro
   435 440 445
   Ile Thr Lys Tyr Ile Met Thr Cys Met Ser Ala Asp Leu Glu Val Val
   450 455 460
   Thr
   465
<210> 18
   <211> 1395
   <212> DNA
   <213> Hepatitis C virus
<400> 18
   gtggacttta tccctgtgga gaacctaggg acaaccatga gatccccggt gttcacggac 60
   aacccctctc caccagcagt gccccagagc ttccaggtgg cccacctgca tgctcccacc 120
   ggcagcggta agagcaccaa ggtcccggct gcgtacgcag cccagggcta caaggtgttg 180
   gtgctcaacc cctctgttgc tgcaacgctg ggctttggtg cttacatgtc caaggcccat 240
   ggggtcgatc ctaatatcag gaccggggtg agaacaatta ccactggcag ccccatcacg 300
   tactccacct acggcaagtt ccttgccgac ggcgggtgct caggaggcgc ttatgacata 360
   ataatttgtg acgagtgcca ctccacggat gccacatcca tcttgggcat cggcactgtc 420
   cttgaccaag cagagactgc gggggcgaga ttggttgtgc tcgccactgc tacccctccg 480
   ggctccgtca ctgtgtccca tcctaacatc gaggaggttg ctctgtccac caccggagag 540
   atcccttttt acggcaaggc tatccccctc gaggtgatca aggggggaag acatctcatc 600
   ttctgtcact caaagaagaa gtgcgacgag ctcgccgcga agctggtcgc attgggcatc 660
   aatgccgtgg cctactaccg cggacttgac gtgtctgtca tcccgaccag cggcgatgtt 720
   gtcgtcgtgt cgaccgatgc tctcatgact ggctttaccg gcgacttcga ctctgtgata 780
   gactgcaaca cgtgtgtcac tcagacagtc gatttcagcc ttgaccctac ctttaccatt 840
   gagacaacca cgctccccca ggatgctgtc tccaggactc agcgccgggg caggactggc 900
   agggggaagc caggcatcta cagatttgtg gcaccggggg agcgcccctc cggcatgttc 960
   gactcgtccg tcctctgtga gtgctatgac gcgggctgtg cttggtatga gctcacgccc 1020
   gccgagacta cagttaggct acgagcgtac atgaacaccc cggggcttcc cgtgtgccag 1080
   gaccatcttg aattttggga gggcgtcttt acgggcctca cccatataga tgcccacttt 1140
   ctatcccaga caaagcagag tggggagaac tttccttacc tggtagcgta ccaagccacc 1200
   gtgtgcgcta gggctcaagc ccctccccca tcgtgggacc agatgtggaa gtgtttgatc 1260
   cgccttaaac ccaccctcca tgggccaaca cccctgctat acagactggg cgctgttcag 1320
   aatgaagtca ccctgacgca cccaatcacc aaatacatca tgacatgcat gtcggccgac 1380
   ctggaggtcg tcacg 1395
<210> 19
   <211> 177
   <212> PRT
   <213> Human immunodeficiency virus
<400> 19
   Glu Asn Arg Trp Gln Val Met Ile Val Trp Gln Val Asp Arg Met Arg
   1 5 10 15
   Ile Asn Thr Trp Lys Arg Leu Val Lys His His Met Tyr Ile Ser Arg
   20 25 30
   Lys Ala Lys Asp Trp Phe Tyr Arg His His Tyr Glu Ser Thr Asn Pro
   35 40 45
   Lys Ile Ser Ser Glu Val His Ile Pro Leu Gly Asp Ala Lys Leu Val
   50 55 60
   Ile Thr Thr Tyr Trp Gly Leu His Thr Gly Glu Arg Asp Trp His Leu
   65 70 75 80
   Gly Gln Gly Val Ser Ile Glu Trp Arg Lys Lys Arg Tyr Ser Thr Gln
   85 90 95
   Val Asp Pro Asp Leu Ala Asp Gln Leu Ile His Leu His Tyr Phe Asp
   100 105 110
   Cys Phe Ser Glu Ser Ala Ile Arg Asn Thr Ile Leu Gly Arg Ile Val
   115 120 125
   Ser Pro Arg Cys Glu Tyr Gln Ala Gly His Asn Lys Val Gly Ser Leu
               130                         135                         140
   Gln Tyr Leu Ala Leu Ala Ala Leu Ile Lys Pro Lys Gln Ile Lys Pro
   145                 150                 155                 160
   Pro Leu Pro Ser Val Arg Lys Leu Thr Glu Asp Arg Trp Asn Lys Pro
                   165                 170                 175
   Gln
<210> 20
   <211> 516
   <212> DNA
   <213> Human immunodeficiency virus
<400> 20
   gaaaacagat ggcaggtgat gattgtgtgg caagtagaca ggatgaggat taacacatgg    60
   aaaagattag taaaacacca tatgtatatt tcaaggaaag ctaaggactg gttttataga   120
   catcactatg aaagtactaa tccaaaaata agttcagaag tacacatccc actaggggat   180
   gctaaattag taataacaac atattggggt ctgcatacag gagaaagaga ctggcatttg   240
   ggtcagggag tctccataga atggaggaaa aagagatata gcacacaagt agaccctgac   300
   ctagcagacc aactaattca tctgcactat tttgattgtt tttcagaatc tgctataaga   360
   aataccatat taggacgtat agttagtcct aggtgtgaat atcaagcagg acataacaag   420
   gtaggatctc tacagtactt ggcactagca gcattaataa aaccaaaaca gataaagcca   480
   cctttgccta gtgttaggaa actgacagag gacaga                             516